(19) Europäisches Patentamt
European Patent Office
Office européen des brevets

(11) EP 4 603 083 A1

(12) EUROPEAN PATENT APPLICATION
published in accordance with Art. 153(4) EPC

(43) Date of publication:
**20.08.2025 Bulletin 2025/34**

(21) Application number: **23876730.5**

(22) Date of filing: **11.10.2023**

(51) International Patent Classification (IPC):
***A61K 9/127*** *(2025.01)* ***A61K 31/496*** *(2006.01)*
***A61P 35/00*** *(2006.01)*

(52) Cooperative Patent Classification (CPC):
**A61K 9/107; A61K 9/127; A61K 31/496;
A61K 47/10; A61K 47/14; A61P 35/00; A61P 35/02**

(86) International application number:
**PCT/CN2023/124000**

(87) International publication number:
**WO 2024/078529 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.10.2022 CN 202211244246**

(71) Applicant: **Wanchun Biotechnology Limited
Florham Park, NJ 07932 (US)**

(72) Inventors:
- **HUANG, Lan
Dalian, Liaoning 116620 (CN)**
- **HE, Chengjiang
Dalian, Liaoning 116620 (CN)**
- **ZHANG, Hao
Dalian, Liaoning 116620 (CN)**

(74) Representative: **Lavoix
Bayerstraße 83
80335 München (DE)**

# (54) PLINABULIN MICELLE COMPOSITION AND PREPARATION METHOD THEREFOR

(57) A Plinabulin micelle composition and a preparation method therefor. Specifically, the Plinabulin micelle composition is essentially composed of Plinabulin, polyoxyl 15-hydroxystearate and propylene glycol, wherein at least part of Plinabulin is encapsulated in the micelle, and can be mixed with a dextrose injection to obtain the Plinabulin micelle preparation for injection, which is used for the clinical treatment of tumor diseases. Both the Plinabulin micelle composition and the micelle preparation have an excellent electrochemical stability, dilution stability and storage stability.

EP 4 603 083 A1

## Description

### Technical Field

The present invention relates to the field of pharmaceutical preparation, specifically to a Plinabulin micelle composition and preparation method thereof.

### Background

Plinabulin ((3Z,6Z)-3-[(5-tert-butyl-1H-imidazol-4-yl)methylene]-6-(phenylmethylene)-2,5-piperazinedione) is a synthetic analog of diketopiperazine phenylahistin (halimide), discovered from marine and terrestrial strains of Aspergillus species, with the following structure:

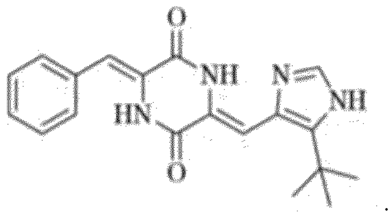

Plinabulin is different in structure from colchicine and its combretastatin-like analogs (e.g., combretastatin phosphate) and binds at or near the colchicine binding site on tubulin monomer. Previous studies have shown that Plinabulin, compared to colchicine, induces vascular endothelial cell tubulin depolymerization and monolayer permeability at low concentration, and induces apoptosis in Jurkat leukemia cells. Studies of Plinabulin as a single agent in patients with advanced malignancies (lung cancer, prostate cancer, and colon cancer) have demonstrated favorable pharmacokinetic, pharmacodynamic, and safety characteristics. However, there remains a need to provide an injectable formulation of Plinabulin with appropriate physicochemical properties.

### Summary of the Invention

The objective of the present invention is to provide a novel Plinabulin micelle composition and preparation method thereof. The Plinabulin micelle has good stability, enhances the water solubility of Plinabulin, delays drug release, extends biological half-life, and has good clinical application value.

The first aspect of the present invention provides a method for preparing a Plinabulin micelle composition comprising the steps of:

s1) mixing poly(ethylene glycol) 15-hydroxystearate with propylene glycol at 35°C-65°C to prepare a clear mixed solution;

s2) mixing the clear mixed liquid from step 1) with Plinabulin at 35°C-65°C and stirring to obtain the Plinabulin micelle composition.

In another preferred embodiment, the weight ratio of poly(ethylene glycol) 15-hydroxystearate to propylene glycol is 1:5-5: 1, preferably 1:3-3: 1, more preferably 2:3.

In another preferred embodiment, the weight ratio of poly(ethylene glycol) 15-hydroxystearate to propylene glycol is 1:9, 2:8, 3:7, 4:6, 5:5, 6:4, 7:3, 8:2, or 9: 1.

In another preferred embodiment, the micelle composition consists essentially of Plinabulin, poly(ethylene glycol) 15-hydroxystearate and propylene glycol.

In another preferred embodiment, in step s1), the temperature is 40°C-60°C.

In another preferred embodiment, in step s2), the temperature is 40°C-60°C.

In another preferred embodiment, step s1) comprises melting poly(ethylene glycol) 15-hydroxystearate at 50°C ±5°C, then adding propylene glycol, maintaining at 40°C±5°C, stirring (e.g., 5min-2h, preferably 20-40min, more preferably 30min) to prepare a clear mixed solution.

In another preferred embodiment, step s1) comprises melting poly(ethylene glycol) 15-hydroxystearate at 35°C-65°C, then adding propylene glycol, maintaining at 35°C-65°C, stirring (e.g., 5min-2h, preferably 10-40min, more preferably 20-30min) to prepare a clear mixed solution.

In another preferred embodiment, step s1) comprises melting poly(ethylene glycol) 15-hydroxystearate at 35°C-65°C, adding it to propylene glycol, maintaining at 35°C-65°C, stirring (e.g., 5min-2h, preferably 10-40min, more

preferably 20-30min) to prepare a clear mixed solution.

In another preferred embodiment, in step s2), the clear mixed solution is added in batches, preferably in 2-5 batches.

In another preferred embodiment, the concentration of Plinabulin is 0.02 mg/ml-4 mg/ml, based on the total weight of the Plinabulin micelle composition.

In another preferred embodiment, the Plinabulin is Plinabulin anhydrous or Plinabulin monohydrate.

In another preferred embodiment, the Plinabulin is Plinabulin anhydrous (crystal form III) or Plinabulin monohydrate (crystal form I).

In another preferred embodiment, in step (s2), the Plinabulin used is Plinabulin monohydrate.

In another preferred embodiment, the Plinabulin is Plinabulin monohydrate (crystal form I).

In another preferred embodiment, the Plinabulin anhydrous is the Plinabulin anhydrous form described in CN113735834B.

In another preferred embodiment, the Plinabulin monohydrate is the Plinabulin monohydrate described in CN113735834B.

In another preferred embodiment, the particle size D50 of the micelles in the resulting Plinabulin micelle composition is 5-100nm.

The second aspect of the present invention provides a Plinabulin micelle composition, wherein the micelle composition comprises a clear mixed solution of Plinabulin, poly(ethylene glycol) 15-hydroxystearate and propylene glycol, wherein the Plinabulin micelle composition is a yellow clear transparent solution with micelle particle size range of 10-100nm.

In another preferred embodiment, the micelle particle size range of the Plinabulin micelle composition is 80-110nm, preferably 90-110nm, for example 100-110nm.

In another preferred embodiment, D50 of the micelle of the Plinabulin micelle composition is 80-110nm, preferably 90-110nm, for example 100-105nm.

In another preferred embodiment, D90 of the micelle of the Plinabulin micelle composition is 100-170nm, preferably 110-160nm, for example 120-150nm.

In another preferred embodiment, in the Plinabulin micelle composition, the concentration of Plinabulin is 1 mg/ml-10 mg/ml, based on the total weight of the Plinabulin micelle composition, preferably the concentration of Plinabulin is 2 mg/ml-5 mg/ml, more preferably the concentration of Plinabulin is 3 mg/ml-4 mg/ml.

In another preferred embodiment, in the Plinabulin micelle composition, the Plinabulin is Plinabulin monohydrate.

In another preferred example, in the Plinabulin micelle composition, the weight ratio of poly(ethylene glycol) 15-hydroxystearate to propylene glycol is 1:5-5:1, preferably 1:3-3:1, more preferably 2:3.

In another preferred example, the Plinabulin micelle composition is prepared by the method as described in the first aspect.

In another preferred example, the Plinabulin micelle composition is sterile.

The third aspect of the present invention provides a liquid injectable Plinabulin composition, comprising:

a D5W solution (5% dextrose injection) of Plinabulin, propylene glycol, and poly(ethylene glycol) 15-hydroxystearate; wherein the volume ratio of propylene glycol to D5W in the composition is about 6:50 to about 6:500.

In another preferred example, in the liquid injectable Plinabulin composition, the Plinabulin is Plinabulin monohydrate.

In another preferred example, the Plinabulin is encapsulated in micelles, preferably, at least 90% of the Plinabulin is encapsulated in micelles.

In another preferred example, at least 95% of the Plinabulin is encapsulated in micelles, preferably at least 97%, more preferably at least 99%.

In another preferred example, the volume ratio of propylene glycol to D5W in the composition is about 6:100 to about 6:400, preferably about 6:150 to about 6:250, more preferably about 6:200.

In another preferred example, the particle size D50 of the micelles in the resulting injectable Plinabulin composition is 5-50nm, preferably 10-30nm.

In another preferred example, the concentration of Plinabulin is about 0.08 mg/ml to about 0.4 mg/ml.

In another preferred example, the volume ratio of poly(ethylene glycol) 15-hydroxystearate to D5W in the liquid injectable Plinabulin formulation is about 4:50-about 4:500, preferably about 4:100 to about 4:500, more preferably about 4:100-about 4:400, for example about 4:100-about 4:300, or about 4:150-about 4:250.

In another preferred example, in the liquid injectable Plinabulin composition, the weight ratio of poly(ethylene glycol) 15-hydroxystearate to propylene glycol is 1:9, 2:8, 3:7, 4:6, 5:5, 6:4, 7:3, 8:2, or 9:1. In another preferred example, the ratio of propylene glycol to poly(ethylene glycol) 15-hydroxystearate is about 60:40 (wt:wt).

In another preferred example, the total amount of impurities contained in the liquid injectable Plinabulin composition is less than 0.5%. In another preferred example, the total amount of impurities contained in the liquid injectable Plinabulin composition is less than 0.1%.
In another preferred example, the liquid injectable Plinabulin composition can be stably stored for about 8 hours to about 12 hours.
In another preferred example, the temperature at which the liquid injectable Plinabulin composition can be stably stored is 10°C to about 37°C, preferably at room temperature.
In another preferred example, at least a portion of the Plinabulin is encapsulated in micelles, preferably, at least 90% of the Plinabulin is encapsulated in micelles.
In another preferred example, greater than about 90% of the Plinabulin in the liquid injectable Plinabulin composition is encapsulated in micelles.
In another preferred example, greater than about 93% of the Plinabulin in the liquid injectable Plinabulin composition is encapsulated in micelles, preferably, greater than about 94% of the Plinabulin is encapsulated in micelles, preferably, greater than about 95% of the Plinabulin is encapsulated in micelles, preferably, greater than about 96% of the Plinabulin is encapsulated in micelles, preferably, greater than about 97% of the Plinabulin is encapsulated in micelles, preferably, greater than about 99% of the Plinabulin is encapsulated in micelles.
In another preferred example, the liquid injectable Plinabulin composition is prepared by the method as described in the fourth aspect of the present invention.
The fourth aspect of the present invention provides a method for preparing a liquid injectable Plinabulin composition, wherein the method comprises:

providing an initial liquid formulation comprising Plinabulin, propylene glycol, and poly(ethylene glycol) 15-hydroxystearate; and
diluting the initial liquid formulation in D5W at a dilution ratio of about 1:5 to about 1:50.

In another preferred example, the initial liquid formulation is the Plinabulin micelle composition as described in the second aspect of the present invention.
In another preferred example, the dilution ratio is about 1:10 to about 1:50, preferably, the dilution ratio is about 1:13 to about 1:30, more preferably, the dilution ratio is about 1:20.
In another preferred example, the initial liquid formulation comprises Plinabulin at a concentration of about 1 mg/ml to about 6 mg/ml, preferably comprises Plinabulin at a concentration of about 3 mg/ml to about 5 mg/ml, more preferably comprises Plinabulin at a concentration of about 4 mg/ml.
In another preferred example, comprising stirring the composition after diluting the initial liquid formulation in D5W, preferably, stirring for at least 5 minutes.
In another preferred example, in the liquid injectable Plinabulin composition, the concentration of Plinabulin is about 0.08 mg/ml to about 0.4 mg/ml.
In another preferred example, the ratio of propylene glycol to poly(ethylene glycol) 15-hydroxystearate in the liquid injectable Plinabulin composition is about 60:40 (wt:wt).
In another preferred example, the initial liquid formulation is the Plinabulin micelle composition as described above.
In another preferred example, the particle size range of micelles in the liquid injectable Plinabulin composition is 90-200nm, preferably 100-150nm, more preferably 100-110nm.
The fifth aspect of the present invention provides use of the Plinabulin micelle composition as described in the second aspect or the liquid injectable Plinabulin composition as described in the third aspect in the preparation of a medicament for preventing and/or treating tumor.
In another preferred example, the tumor is selected from: lung cancer (such as small cell lung cancer, non-small cell lung cancer), prostate cancer, colon cancer, brain tumor (such as glioblastoma, polymorphic glioblastoma, giant cell glioblastoma, metastatic brain tumor), head and neck cancer, gastric cancer, pancreatic cancer, breast cancer, renal cancer, bladder cancer, ovarian cancer, cervical cancer, melanoma, myeloma, lymphoma or leukemia.
It should be understood that, within the scope of the present invention, the above-mentioned technical features of the present invention and the technical features specifically described below (such as in the examples) can be combined with each other to form new or preferred technical solutions. Due to space limitations, these combinations will not be enumerated one by one.

## Brief Description of the Drawings

Figure 1 shows the Tyndall effect of Plinabulin micelle composition.
Figure 2 shows the Tyndall effect of liquid injectable Plinabulin composition.

**Detailed Description of the Invention**

After extensive and in-depth research, the inventors of the present invention have unexpectedly obtained a novel Plinabulin micelle composition with excellent stability. The Plinabulin micelle composition can improve the water solubility of Plinabulin and exhibits excellent electrochemical stability, dilution stability, and storage stability.

Specifically, the present invention uses Plinabulin monohydrate and a specific ratio of propylene glycol and poly(ethylene glycol) 15-hydroxystearate to unexpectedly prepare a Plinabulin micelle composition, wherein Plinabulin is encapsulated in micelles. This micelle composition possesses excellent stability, electrochemical stability, dilution stability, and storage stability.

Based on the obtained Plinabulin micelle composition, a liquid injectable Plinabulin composition is prepared by dilution with D5W solution. After dilution, the Plinabulin micelle composition still exists with Plinabulin encapsulated therein. This diluted injectable solution has excellent infusion stability, storage stability, high encapsulation rate of Plinabulin, and low impurity content, making it suitable as an injectable formulation of Plinabulin.

On this basis, the present invention is completed.

The present invention discloses Plinabulin micellar encapsulation composition and method for preparing and using Plinabulin composition. Plinabulin, (3Z,6Z)-3-[(5-tert-butyl-1H-imidazol-4-yl)methylene]-6-( phenylmethylene)-2,5-piperazinedione), is a synthetic analog of the natural compound diketopiperazine phenylahistin. Plinabulin can be readily prepared according to the methods and steps detailed in U.S. Patent No. 7,064,201 and 7,919,497, the entire contents of which are incorporated herein by reference.

Unless otherwise defined, all technical and scientific terms used herein have the same meaning as commonly understood by one of ordinary skill in the art to which this invention belongs. Although any methods and materials similar or equivalent to those described herein can be used in the practice or testing of the present invention, preferred methods and materials are described herein.

**Terms**

Unless otherwise defined, all technical and scientific terms used herein have the same meaning as commonly understood by one of ordinary skill in the art to which this disclosure belongs. All patents, applications, published applications, and other publications are incorporated by reference in their entirety. If a term is defined in multiple ways, the definition in this section prevails unless otherwise indicated.

As used herein, Plinabulin includes crystalline forms or amorphous forms, such as Plinabulin monohydrate, anhydrous Plinabulin, and Plinabulin solvate. The monohydrate is preferred.

In the present invention, "Plinabulin injectable micellar formulation," "liquid injectable Plinabulin composition," "liquid injectable formulation," "liquid injectable Plinabulin formulation," and "liquid Plinabulin composition for injection" have the same meaning and can be used interchangeably.

The term "agent" is used herein to denote a compound, a mixture of compounds, a biological macromolecule, or an extract made from biological materials.

The terms "cancer", "neoplasm" and "carcinoma" used interchangeably herein, refer to cells that exhibit relatively autonomous growth, thus exhibiting an abnormal growth phenotype characterized by a significant loss of control of cell proliferation. In general, cells of interest for detection or treatment in the present application include premalignant (e.g., benign), malignant, pre-metastatic, metastatic, and non-metastatic cells. Detection of cancer cells is particularly significant.

The term "subject" as used herein refers to a human or non-human mammal, such as a dog, cat, mouse, cow, sheep, pig, goat, non-human primate, or bird, such as a chicken, and any other vertebrate or invertebrate.

The term "mammal" is used in its usual biological sense. Thus, it specifically includes but is not limited to primates, including apes (chimpanzees, gibbons, monkeys) and humans, cattle, horses, sheep, goats, pigs, rabbits, dogs, cats, rodents, rats, mice, guinea pigs, or similar animals.

The terms "effective amount" or "therapeutically effective amount" as used herein refer to an amount of a therapeutic agent that is effective to some extent in alleviating one or more symptoms of a disease or condition, or reducing the likelihood of occurrence, and may include an amount that cures the disease or condition.

The term "treat" as used herein refers to administering a compound or pharmaceutical composition to a subject for preventative and/or therapeutic purposes. The term "preventative treatment" refers to treating a subject who has not yet exhibited symptoms of a disease or condition but is susceptible or otherwise at risk for a particular disease or condition, whereby the treatment reduces the likelihood that the patient will develop the disease or condition. The term "therapeutic treatment" refers to treating a subject who already has a disease or condition.

## Formulations

In some embodiments, Plinabulin is provided in the form of a concentrated liquid formulation, which can then be prepared into a diluted liquid injection formulation through dilution. In some embodiments, the concentrated liquid formulation includes one or more solvents, which may include poly(ethylene glycol) 15-hydroxystearate and/or propylene glycol. In some embodiments, the one or more solvents include at least 30% (by weight) of poly(ethylene glycol) 15-hydroxystearate, at least 35% (by weight) of poly(ethylene glycol) 15-hydroxystearate, at least 40% of poly(ethylene glycol) 15-hydroxystearate, at least 45% (by weight) of poly(ethylene glycol) 15-hydroxystearate, or include and/or span a range of the above values. In some embodiments, the one or more solvents include at least 50% (by weight) of propylene glycol, at least 55% (by weight) of propylene glycol, at least 50% (by weight) of propylene glycol, at least 55% (by weight) of propylene glycol, at least 60% (by weight) of propylene glycol, or include and/or span a range of the above values. In some embodiments, the one or more solvents include 40% (by weight) poly(ethylene glycol) 15-hydroxystearate and 60% (by weight) propylene glycol. In some embodiments, the ratio of propylene glycol to poly(ethylene glycol) 15-hydroxystearate in the liquid injection formulation is about 60:40 (by weight).

In some embodiments, the concentrated liquid formulation is a micelle composition. Plinabulin is encapsulated in micelles. In some embodiments, the components of the micelle composition include poly(ethylene glycol) 15-hydroxystearate, propylene glycol, and Plinabulin, wherein Plinabulin is encapsulated in micelles. In some embodiments, at least 60%, at least 62%, at least 64%, at least 66%, at least 68%, at least 70%, at least 72%, at least 74%, at least 76%, at least 78%, at least 80%, at least 82%, at least 84%, at least 86%, at least 88%, at least 90%, at least 92%, at least 93%, at least 94%, at least 95%, at least 96%, at least 97%, at least 98%, at least 99%, or a range including and/or spanning the above values of Plinabulin is encapsulated in micelles.

In some embodiments, the Plinabulin in the micelle composition is Plinabulin monohydrate. When the anhydrous form of Plinabulin is used, the resulting micelle composition has poor solubility.

The concentration of Plinabulin in the concentrated liquid formulation can be between 1mg/ml and 10mg/ml, between 2mg/ml and 6mg/ml, or about 4mg/ml.

In some embodiments, the concentrated liquid formulation can be diluted with a diluent to form a diluted liquid injection formulation. In some embodiments, the diluent is water. In some embodiments, the diluent is saline. In some embodiments, the diluent is an aqueous dextrose solution. The concentration of dextrose can be 1% to 20%, 2% to 10%, or about 5% (i.e., DSW).

In some embodiments, the diluted liquid injection formulation includes poly(ethylene glycol) 15-hydroxystearate, propylene glycol, and D5W (i.e., dextrose and water). In some embodiments, the volume ratio of propylene glycol to D5W in the liquid injectable Plinabulin formulation ranges from about 6:50 to about 6:500, about 6:50 to about 6:450, about 6:50 to about 6:400, about 6:50 to about 6:350, about 6:50 to about 6:300, about 6:50 to about 6:250, about 6:50 to about 6:200, about 6:50 to about 6:150, about 6:50 to about 6:100, about 6:100 to about 6:500, about 6:100 to 6:400, about 6:150 to about 6:250, or about 6:200. In some embodiments, the volume ratio of propylene glycol to D5W in the liquid injectable Plinabulin formulation is about 6:70. In some embodiments, the volume ratio of propylene glycol to D5W in the liquid injectable Plinabulin formulation is about 6:140. In some embodiments, the volume ratio of propylene glycol to D5W in the liquid injectable Plinabulin formulation is about 6:400.

In some embodiments, the volume ratio of poly(ethylene glycol) 15-hydroxystearate to D5W in the liquid injectable Plinabulin formulation ranges from about 4:50 to about 4:500, about 4:100 to about 4:500, about 4:100 to about 4:400, about 4:100 to about 4:300, about 4:150 to about 4:250, or about 4:200.

In some embodiments, the Plinabulin in the liquid injectable Plinabulin formulation (composition) is Plinabulin monohydrate.

In some embodiments, the concentration of Plinabulin in the liquid injectable Plinabulin formulation (composition) is about 0.02 mg/ml, 0.03mg/ml, 0.04mg/ml, 0.05mg/ml, 0.06mg/ml, 0.07mg/ml, 0.08mg/ml, 0.09mg/ml, 0.1mg/ml, 0.11 mg/ml, 0.12 mg/ml, 0.13 mg/ml, 0.14 mg/ml, 0.15 mg/ml, 0.16 mg/ml, 0.17 mg/ml, 0.18 mg/ml, 0.19 mg/ml, 0.2 mg/ml, 0.3 mg/ml, 0.4 mg/ml, 0.5 mg/ml, 1 mg/ml, 2 mg/ml, 3 mg/ml, 4 mg/ml, or includes and/or spans a range of the above values. In some embodiments, in the liquid injectable Plinabulin composition, the concentration of Plinabulin is about 0.08mg/ml to about 0.4 mg/ml. In some embodiments, in the liquid injectable Plinabulin composition, the concentration of Plinabulin is about 0.1 mg/ml to about 0.3 mg/ml. In some embodiments, in the liquid inj ectable Plinabulin composition, the concentration of Plinabulin is about 0.2 mg/ml.

In some embodiments, the liquid injectable Plinabulin formulation (composition) contains impurities less than 5%, less than 4%, less than 3%, less than 2%, less than 1%, less than 0.5%, or includes and/or spans a range of the above values. In some embodiments, the liquid injectable Plinabulin formulation (composition) contains ether impurities less than 0.5%. In some embodiments, the liquid injectable Plinabulin formulation (composition) contains alcohol impurities less than 0.5%. In some embodiments, the liquid injectable Plinabulin formulation (composition) contains water less than 0.5%.

In some embodiments, the liquid injectable Plinabulin formulation (composition) can be stably stored for about 4 hours, 5 hours, 6 hours, 7 hours, 8 hours, 9 hours, 10 hours, 11 hours, 12 hours, 13 hours, 14 hours, 15 hours, 16 hours, 17

hours, 18 hours, 19 hours, 20 hours, 21 hours, 22 hours, 23 hours, 24 hours, 36 hours, 48 hours, or ranges including and/or spanning the aforementioned values.

In some embodiments, the liquid injectable Plinabulin formulation (composition) can be stably stored at 10°C, 12°C, 14°C, 16°C, 18°C, 20°C, 22°C, 24°C, 26°C, 28°C, 30°C, 32°C, 34°C, 36°C, 38°C, 40°C, or ranges including and/or spanning the aforementioned values.

In some embodiments, when the concentrated liquid formulation is diluted with a diluent (such as D5W), a portion of Plinabulin is encapsulated within micelles. In some embodiments, the micelles are formed by interactions between poly(ethylene glycol) 15-hydroxystearate and DSW. In some embodiments, at least 60%, at least 62%, at least 64%, at least 66%, at least 68%, at least 70%, at least 72%, at least 74%, at least 76%, at least 78%, at least 80%, at least 82%, at least 84%, at least 86%, at least 88%, at least 90%, at least 92%, at least 93%, at least 94%, at least 95%, at least 96%, at least 97%, at least 98%, at least 99%, or ranges including and/or spanning the aforementioned values of Plinabulin in the injectable liquid formulation (composition) is encapsulated in micelles.

In some embodiments, the average encapsulation efficiency of the liquid formulation is at least 90%, at least 92%, at least 94%, at least 96%, at least 98%, or ranges including and/or spanning the aforementioned values.

**Preparation Method**

In certain aspects, methods for preparing the liquid injectable formulations (compositions) described herein are disclosed. In some embodiments, a method for preparing a liquid injectable Plinabulin composition comprises providing an initial concentrated liquid formulation comprising Plinabulin, propylene glycol, and poly(ethylene glycol) 15-hydroxystearate (PEG 15-hydroxystearate); and diluting the initial liquid formulation in DSW. In various embodiments, the dilution ratio between the initial concentrated liquid formulation and D5W is about 1:5 to about 1:50, about 1:10 to about 1:50, about 1:10 to about 1:40, about 1:15 to about 1:30, about 1:15 to about 1:25, or about 1:20. In some embodiments, the dilution ratio is about 1:10 to about 1:400. In some embodiments, the dilution ratio is about 1:10 to about 1:300. In some embodiments, the dilution ratio is about 1:10 to about 1:200. In some embodiments, the dilution ratio is about 1:10 to about 1:100. In some embodiments, the dilution ratio is about 1:10 to about 1:80. In some embodiments, the dilution ratio is about 1:10 to about 1:60. In some embodiments, the dilution ratio is about 1:10 to about 1:50. In some embodiments, the dilution ratio is about 1:10 to about 1:40. In some embodiments, the dilution ratio is about 1:10 to about 1:30. In some embodiments, the dilution ratio is about 1:10 to about 1:20. In some embodiments, the dilution ratio is about 1:20 to about 1:400. In some embodiments, the dilution ratio is about 1:20 to about 1:300. In some embodiments, the dilution ratio is about 1:20 to about 1:200. In some embodiments, the dilution ratio is about 1:20 to about 1:100. In some embodiments, the dilution ratio is about 1:20 to about 1:80. In some embodiments, the dilution ratio is about 1:20 to about 1:60. In some embodiments, the dilution ratio is about 1:20 to about 1:50. In some embodiments, the dilution ratio is about 1:20 to about 1:40. In some embodiments, the dilution ratio is about 1:20 to about 1:30.

In some embodiments, the dilution method comprises introducing (e.g., by injection) the initial concentrated liquid formulation into a container containing a volume of DSW. In some embodiments, the container is an intravenous (IV) bag. In some embodiments, after introducing the initial concentrated liquid formulation into the container, the mixture is agitated. Various agitation methods include hand shaking, manual stirring, or vortexing. In various embodiments, the mixture is agitated for at least 1 minute, at least 2 minutes, at least 3 minutes, at least 5 minutes, at least 10 minutes, at least 20 minutes, at least 30 minutes, at least 45 minutes, at least 60 minutes, at least 90 minutes, or ranges including and/or spanning the aforementioned values.

In some embodiments for preparing the initial concentrated liquid formulation, propylene glycol is added first, followed by PEG15-hydroxystearate, and then Plinabulin. In some embodiments, PEG15-hydroxystearate is added first, followed by propylene glycol, and then Plinabulin. In some embodiments, the initial concentrated liquid formulation is mixed at room temperature.

In some embodiments for preparing the initial concentrated liquid formulation, propylene glycol is heated to about 25°C and maintained at about 25°C during the addition of PEG15-hydroxystearate. In some embodiments, Plinabulin is added to the PEG15-hydroxystearate and propylene glycol and mixed at about 25°C.

In some embodiments for preparing the initial concentrated liquid formulation, propylene glycol is heated to about 40°C and maintained at about 40°C during the addition of PEG15-hydroxystearate. In some embodiments, Plinabulin is added to the PEG15-hydroxystearate and propylene glycol and mixed at about 40°C.

In some embodiments for preparing the initial concentrated liquid formulation, PEG15-hydroxystearate are heated to about 25°C and maintained at about 25°C during the addition of propylene glycol. In some embodiments, Plinabulin is added to the PEG15-hydroxystearate and propylene glycol and mixed at about 25°C.

In some embodiments for preparing the initial concentrated liquid formulation, PEG15-hydroxystearate are heated to about 40°C and maintained at about 40°C during the addition of propylene glycol. In some embodiments, Plinabulin is added to the PEG15-hydroxystearate and propylene glycol and mixed at about 40°C.

In some embodiments of preparing the initial concentrated liquid formulation, poly(ethylene glycol) 15-hydroxystearate is heated to about 60°C and maintained at about 60°C when propylene glycol is added. In some embodiments, Plinabulin is added to the poly(ethylene glycol) 15-hydroxystearate and propylene glycol, and mixed at about 60°C.

Use **and Method**

Some embodiments relate to a method of preventing or reversing cancer progression in a subject. In some embodiments, the method comprises administering to the subject the injectable liquid formulation described herein. Some embodiments relate to a method of inhibiting cancer progression. Further uses of the injectable liquid formulation described herein include those in U.S. Patent Nos. 7,919,497; 10,238,650; 10,155,748; 10,076,518; and 10,596,169; and WO 2016/130839; WO 2017/214052; WO 2018/144764; WO 2018/169887; WO 2019/147615; WO 2019/152530; WO 2020/037285; WO 2021/076485; and WO 2021/225908; all of which are incorporated herein by reference in their entirety.

In some embodiments, the treatment regimen includes administering the injectable liquid formulation described herein once every 1, 2, 3, 4, 5, 6, 7, or 8 weeks. In some embodiments, the treatment regimen includes administering the injectable liquid formulation twice every 1, 2, 3, 4, 5, 6, 7, or 8 weeks. In some embodiments, the treatment regimen includes administering the injectable liquid formulation described herein once weekly within a treatment cycle of 1, 2, 3, 4, 5, 6, 7, or 8 weeks. In some embodiments, the treatment regimen includes administering the injectable liquid formulation twice weekly within a treatment cycle of 1, 2, 3, 4, 5, 6, 7, or 8 weeks. In some embodiments, the treatment regimen includes administering the injectable liquid formulation described herein on days 1, 8, and 15 of a 21-day treatment cycle.

Treatment cycle may be repeated as long as the regimen is clinically tolerable. In some embodiments, the treatment cycle of the injectable liquid formulation described herein is repeated n times, wherein n is an integer in the range of 2 to 30. In some embodiments, n is 2, 3, 4, 5, 6, 7, 8, 9, or 10. In some embodiments, a new treatment cycle may occur immediately after completion of the previous treatment cycle. In some embodiments, a new treatment cycle may occur after a period of time following completion of the previous treatment cycle. In some embodiments, a new treatment cycle may occur 1, 2, 3, 4, 5, 6, or 7 weeks after completion of the previous treatment cycle.

In some embodiments, the method comprises administering Plinabulin at a dose of about 5 $mg/m^2$ to 150 $mg/m^2$. In some embodiments, the administered dose of Plinabulin is greater than 20 $mg/m^2$. In some embodiments, the administered dose of Plinabulin is greater than 30 $mg/m^2$. In some embodiments, the administered dose of Plinabulin is greater than 40 $mg/m^2$.

In some embodiments, the injectable liquid formulation described herein is administered on day 1 of a 14-day administration cycle. In some embodiments, the injectable liquid formulation described herein is administered on day 1 of a 21-day administration cycle.

In some embodiments, the injectable liquid formulation described herein is co-administered with one or more G-CSF drugs.

Some embodiments include a kit comprising one or more containers.

In some embodiments, the container comprises plastic or glass, or a combination thereof, including but not limited to any one or more plastics or glasses used by those skilled in the art in light of the teachings herein.

In some embodiments, the container is a vial. In some embodiments, the vial contains about 1 mg, 2 mg, 5 mg, 10 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, or a range including and/or spanning the aforementioned values of Plinabulin. In some embodiments, the vial contains Plinabulin, propylene glycol, and poly(ethylene glycol) 15-hydroxystearate (PEG15-hydroxystearate). In some embodiments, the vial includes about 4 mg/ml of Plinabulin, propylene glycol, and poly(ethylene glycol) 15-hydroxystearate (at a ratio of about 60:40 (wt:wt)). In some embodiments, the volume of the liquid formulation in the vial is about 10 ml.

In some embodiments, the container is an intravenous injection bag. In some embodiments, the intravenous injection bag includes D5W. In some embodiments, the volume of D5W in the intravenous injection bag is about 50 ml to about 500 ml, about 100 ml to about 500 ml, about 100 ml to about 400 ml, about 100 ml to about 300 ml, about 150 ml to about 250 ml, or about 200 ml.

In some embodiments, the kit includes a vial as described above and an intravenous injector as described above.

**Preparation of Plinabulin Micelle composition**

After melting poly(ethylene glycol) 15-hydroxystearate at 50°C ± 5°C, the prescribed amount of melted poly(ethylene glycol) 15-hydroxystearate is weighed and added to a nitrogen-filled compounding tank, followed by the prescribed amount of propylene glycol, and stirred for 30 minutes while maintaining at 40°C ± 5°C.

An appropriate amount of the mixed solution of poly(ethylene glycol) 15-hydroxystearate and propylene glycol is taken from the compounding tank (DGJ-14) into a beaker and Duran bottle for standby use. The prescribed amount of Plinabulin is weighed in an isolator in the cytotoxic drug weighing room. The mixed solution of poly(ethylene glycol) 15-hydroxystearate and propylene glycol from the beaker is added, stirred for 5 minutes, and then transferred to the compounding tank. The beaker is thoroughly rinsed three times with the mixed solution of poly(ethylene glycol) 15-hydroxystearate and propylene glycol from the Duran bottle and transferred to the compounding tank. The magnetic stirrer is turned on (frequency 40 Hz) and stirred for 1 hour before sampling for intermediate product testing (samples should be protected from light). Throughout this process, the temperature of the drug solution is maintained at 40°C ± 5°C. Intermediate product samples are taken according to the sampling plan and tested for: appearance, moisture content, density, related substances, microbial limits, bacterial endotoxins, and content determination. The time from preparation to sterile filtration should not exceed 6 hours. The Tyndall effect of the Plinabulin micelle composition is shown in Figure 1.

**Injectable Plinabulin Composition**

The Plinabulin micelle composition as described above is diluted in D5W at a dilution ratio of about 1:5 to about 1:50 (wt) to obtain a Plinabulin composition for injection, wherein the Tyndall effect diagram of the 1:20 dilution ratio is shown in Figure 2.

**Compared with the prior art, this application has the following main advantages:**

1. The Plinabulin micelle composition of this application has excellent dilution stability.
2. The Plinabulin micelle composition of this application, and especially the prepared Plinabulin composition for injection, has excellent storage stability.

The invention will be further illustrated with reference to specific examples below. It should be understood that these examples are only used to illustrate the invention and not to limit the scope of the invention. Unless specifically stated otherwise, experimental methods for which specific conditions are not indicated in the following examples are generally carried out under conventional conditions or as recommended by the manufacturer. Unless otherwise specified, percentages and parts are by weight.

**Example 1: Effect of different formulation addition sequences on the dissolution rate of Plinabulin and the particle size distribution of the final product.**

**1. Different formulation addition sequences (propylene glycol/poly(ethylene glycol) 15-hydroxystearate: 6/4)**

Table 1-1 Component of the composition

| Component | Amount/g | Remarks |
|---|---|---|
| Propylene glycol | 30.9 | ratio of propylene glycol to poly(ethylene glycol) 15-hydroxystearate: 6/4 |
| Poly(ethylene glycol) 15-hydroxystearate | 20.6 | |
| Active pharmaceutical ingredient | 0.2 | drug concentration: 4 mg/mL |
| Total volume: 50mL | | |

**1.1 First add propylene glycol, then poly(ethylene glycol) 15-hydroxystearate, and finally the drug**

Poly(ethylene glycol) 15-hydroxystearate at 25 °C (solid state), 40 °C (molted) and 60 °C were added to propylene glycol respectively, followed by the addition of Plinabulin to prepare Plinabulin micelle compositions A, B and C, which were then diluted with 5% dextrose injection at 1:20 to obtain liquid Plinabulin compositions for injection (formulations). Particle size was determined. The results showed that: under the preparation process conditions of adding propylene glycol first, then poly(ethylene glycol) 15-hydroxystearate, and finally the drug, the dissolution rate of the active pharmaceutical ingredient increased with increasing temperature. At 25 °C, it took nearly 2h for the drug to dissolve, while at 40 °C and 60 °C, the active pharmaceutical ingredient was completely dissolved in about 10 min. The average particle size of Plinabulin micelle compositions A and B was about 100 nm, while Plinabulin micelle composition C could not be analyzed for particle size. The particle size distributions after 1:20 dilution of different Plinabulin micelle compositions were similar, indicating that the preparation temperature had little effect on the particle size distribution after dilution.

### 1.2 First add poly(ethylene glycol) 15-hydroxystearate, then propylene glycol, and finally the drug

Propylene glycol was added to poly(ethylene glycol) 15-hydroxystearate at 25°C (solid state), 40°C (molted) and 60°C respectively, followed by the addition of Plinabulin to prepare Plinabulin micelle compositions D, E and F, which were then diluted with 5% dextrose injection at 1:20 to obtain liquid Plinabulin compositions for injection (formulations). Particle size was determined. The results showed that: under the conditions of adding poly(ethylene glycol) 15-hydroxystearate first, then propylene glycol, and finally the drug, the dissolution rate of the drug increased with increasing temperature. At 25°C, the drug was still not dissolved after 5h of stirring; at 40°C and 60°C, the drug was completely dissolved within 15min. Particle size distribution determination results showed that the preparation temperature had almost no effect on the particle distribution of the micelle composition, and the addition sequence of poly(ethylene glycol) 15-hydroxystearate and propylene glycol did not affect the particle size of the formulation.

### 1.3 First add the drug, then propylene glycol, and finally poly(ethylene glycol) 15-hydroxystearate

The active pharmaceutical ingredient was added first, followed by propylene glycol, and then poly(ethylene glycol) 15-hydroxystearate at 25°C (solid state), 40°C (molted) and 60°C respectively to prepare Plinabulin micelle compositions G, H and I, which were then diluted with 5% dextrose injection at 1:20 to obtain liquid Plinabulin compositions for injection (formulations). Particle size was determined. The results showed that: when the temperature was not lower than 40°C, the active pharmaceutical ingredient was dissolved in the propylene glycol solvent alone in a relatively short time, and the solution remained clear after adding poly(ethylene glycol) 15-hydroxystearate; but when the temperature of propylene glycol was 25°C, the active pharmaceutical ingredient was difficult to dissolve. Particle size distribution determination results showed that the preparation method of dissolving the drug in propylene glycol first had no effect on the particle size distribution.

### 1.4 First add the drug, then a mixed solution of propylene glycol and poly(ethylene glycol) 15-hydroxystearate

The active pharmaceutical ingredient was added first, followed by a solution of propylene glycol and poly(ethylene glycol) 15-hydroxystearate pre-mixed at 40°C to prepare Plinabulin micelle composition J, which was then diluted with 5% dextrose injection at 1:20 to obtain a liquid Plinabulin composition for injection (formulation). Particle size was determined. The results showed that: compared with the micelle composition particle size results in 1.1, the formulation prepared by adding the active pharmaceutical ingredient first and then the mixed solution of propylene glycol and poly(ethylene glycol) 15-hydroxystearate had an increased average particle size from 104.61nm to 112.52nm, and D10 increased significantly. However, there was no significant difference in the particle size of its diluted samples.

### 1.5 First add the drug, then poly(ethylene glycol) 15-hydroxystearate, and finally propylene glycol

The active pharmaceutical ingredient was added first, followed by the poly(ethylene glycol) 15-hydroxystearate that has been melted at 40°C, maintained the temperature at 40°C and stirred, and then propylene glycol was added to prepare the Plinabulin micelle composition H, which was then diluted with 5% dextrose injection at 1:20 to obtain a liquid Plinabulin composition for injection (formulation). Particle size was determined. The results showed that: the active pharmaceutical ingredient was not dissolved in poly(ethylene glycol) 15-hydroxystearate, and after adding propylene glycol, the dissolution time of the active pharmaceutical ingredient increased compared to that in 1.1. The particle size results showed that, compared with the particle size results in 1.1, when the active pharmaceutical ingredient is first dispersed in poly(ethylene glycol) 15-hydroxystearate and then propylene glycol is added, the particle size of the stock solution significantly increased. The average particle size of the micelle composition increased from 104.61nm to 127.06nm, D10 showed no significant change, D50 increased from 100.08nm to 118.03nm, and D90 increased from 145.88nm to 192.41nm. However, there was no significant difference in particle size for the 1:20 diluted samples.

In summary, the preparation time of the micelle composition decreases as the preparation temperature increases. In terms of process flow, when the active pharmaceutical ingredient is added last, the sequence of adding poly(ethylene glycol) 15-hydroxystearate and propylene glycol has no significant effect on the preparation process and the particle size results of the micelle composition. When the active pharmaceutical ingredient is added first, dispersing it in propylene glycol and then adding poly(ethylene glycol) 15-hydroxystearate shortens the preparation time, and its particle size results show no significant difference from the production conditions; adding a mixed solution of poly(ethylene glycol) 15-hydroxystearate and propylene glycol shows no significant difference in preparation time and phenomena, but the particle size distribution results of the micelle composition show increases in average particle size, D10, D50, and D90; dispersing first in poly(ethylene glycol) 15-hydroxystearate and then adding propylene glycol slightly increases the preparation time, and the particle size distribution results of the micelle composition show increases in average particle size, D10, D50, and D90, which are greater than those of the samples obtained from the addition of the mixed solution of

poly(ethylene glycol) 15-hydroxystearate and propylene glycol.

**2 Different formulation ratios and temperatures**

**2.1 Propylene glycol: poly(ethylene glycol) 15-hydroxystearate (7:3)**

Table 1-2 Component of the composition

| Component | Amount/g | Remarks |
|---|---|---|
| Propylene glycol | 36.1 | ratio of propylene glycol to poly(ethylene glycol) 15-hydroxystearate: 7/3<br>drug concentration: 4 mg/mL |
| Poly(ethylene glycol) 15-hydroxystearate | 15.4 | |
| Active pharmaceutical ingredient | 0.2 | |
| Total volume: 50mL | | |

Poly(ethylene glycol) 15-hydroxystearate at 40°C (melted) and 60°C were added to ethylene glycol, respectively, then Plinabulin was added to prepare Plinabulin micelle compositions K and L, which were then diluted with 5% dextrose injection at 1:20 to obtain liquid Plinabulin compositions for injection (formulations). Particle size was determined. The results showed that: when the ratio of propylene glycol to poly(ethylene glycol) 15-hydroxystearate was 7:3, the solution was turbid, and although the active pharmaceutical ingredient was dissolved after addition, the solution remained turbid. The dissolution rate of the active pharmaceutical ingredient still positively correlated with temperature; as the temperature increased, the clarity of the sample solution also increased. Due to the turbidity of the samples and the non-homogeneous system, it was difficult to determine the particle size. Only the formulation prepared at 60°C, after dilution, yielded particle size analysis results, which were similar in size and distribution to those of the formulation prepared in 1.1.

**2.2 Propylene glycol: poly(ethylene glycol) 15-hydroxystearate (3:7)**

Table 1-3 Component of the composition

| Component | Amount/g | Remarks |
|---|---|---|
| Propylene glycol | 15.4 | ratio of propylene glycol/poly(ethylene glycol) 15-hydroxystearate: 3/7<br>drug concentration: 4 mg/mL |
| Poly(ethylene glycol) 15-hydroxystearate | 36.1 | |
| Active pharmaceutical ingredient | 0.2 | |
| Total volume: 50mL | | |

Poly(ethylene glycol) 15-hydroxystearate at 40°C (melted) and 60°C were added to ethylene glycol, respectively, then Plinabulin was added to prepare Plinabulin micelle compositions M and N, which were then diluted with 5% dextrose injection at 1:20 to obtain liquid Plinabulin compositions for injection (formulations). Particle size was determined. The results showed that: the dissolution rate of the active pharmaceutical ingredient increased with increasing temperature. The particle size distribution results showed that when the ratio of propylene glycol to poly(ethylene glycol) 15-hydroxystearate was changed to 3:7, the particle size of the micelle composition significantly decreased.

In summary, when the ratio of propylene glycol to poly(ethylene glycol) 15-hydroxystearate is changed, the properties of the mixed solution change. When the ratio of propylene glycol to poly(ethylene glycol) 15-hydroxystearate is 7:3, the solution is turbid and significantly affects the dissolution rate of the drug; when the ratio of propylene glycol to poly(ethylene glycol) 15-hydroxystearate is 3:7, there is no significant difference in the preparation process, but the particle size is significantly reduced. It is possible that within a certain range, as the surfactant concentration increases, the micelle particle size gradually decreases.

**Example 2**

This example studied the micelle characteristics of the prepared injectable Plinabulin solution. The encapsulation rate of Plinabulin after dilution was determined by separating the drug encapsulated in micelles from the free drug.

Since the target compound to be determined was Plinabulin in the formulation, liquid chromatography was selected as the content determination method for this product, and the reproducibility and specificity of this method were confirmed. The liquid chromatography conditions and the conduct of the experiment were conducted as shown in Table 2-1.

Table 2-1 Liquid chromatography method - Specific parameters

| **Item** | **Content** | | |
|---|---|---|---|
| Column | ACE C18<br>4.6mm×150mm, 5.0μm | | |
| Mobile phase | A: 0.01mol/L phosphate buffer (take 0.82g±0.01 of sodium dihydrogen phosphate monohydrate and 1.20g±0.01g of sodium phosphate, dissolve in water and dilute to 1000ml, mix well.);<br>B: Acetonitrile | | |
| Elution process | **Time(min)** | **Mobile phase A(%)** | **Mobile phase B(%)** |
| | 0 | 0 | 10 |
| | .0 | 0 | 0 |
| | 15.0 | 55 | 45 |
| | 16□0 | 20 | 80 |
| | 17.0 | 0 | 80 |
| | 17.1 | | 10 |
| | 0.0 | 9 | |

Flow rate: 1.2 ml/min; Column temperature: 40 °C; Injector temperature: 20 °C; Detection wavelength: 330 nm; Injection volume: 10 μl

According to the content determination method in Table 2-1, samples of Plinabulin solution were prepared. The reproducibility of the method was determined.

Table 2-2 Solution Preparation Process

| **Item** | **Content** |
|---|---|
| Control solution | 0.2 mg/ml Plinabulin solution in absolute ethanol |
| Test solution | Accurately weigh about 5g of Plinabulin and place it in a 100ml volumetric flask, add absolute ethanol to dissolve and dilute to the mark, mix well. Prepare six samples in parallel. |
| Sensitivity solution | 0.1 μg/ml Plinabulin solution in absolute ethanol |

10μL of Plinabulin solution, control solution, and test solution were injected into the HPLC. Peak areas were calculated and Plinabulin content in the product was determined according to method standards. The measurement results were shown in Tables 2-3 and 2-4.

Table 2-3 Detection Method Confirmation -Reproducibility-System Suitability

| **Solution** | **Item** | **Result** | **Acceptance Criteria** |
|---|---|---|---|
| Sensitivity solution | Signal-to-noise ratio | S/N: 58.76 | S/N greater than 10 |
| control solution (1) | Relative standard deviation of peak area | RSD%: 0.05% | The RSD% of peak area should not be greater than 2.0% for 5 consecutive injections. |
| | Recovery rate | 100.3% | The recovery rates of Plinabulin are all between 98.0% and 102.0% of the theoretical value. |
| control solution (2) | Recovery rate | 99.7% | The recovery rates of Plinabulin should be between 98.0% and 102.0% of the theoretical value. |

Table 2-4 Determination Method Confirmation-Reproducibility-Content Determination

| Test solution | Content (%) | Average content (%) | RSD% | Acceptance Criteria |
|---|---|---|---|---|
| 1 | 97.20 | 96.9 | 0.5 | The % RSD of the content of 6 parallel samples should not be greater than 2.0%. |
| 2 | 96.28 | | | |
| 3 | 96.50 | | | |
| 4 | 97.27 | | | |
| 5 | 97.51 | | | |
| 6 | 96.83 | | | |

The test data and chromatograms showed that the determination method for this product had good sensitivity and reproducibility, which can meet the requirements for determining the encapsulation rate of this diluted preparation.

The equilibrium solubility of the drug in the injected formulation solvent system without solubilizer (poly(ethylene glycol) 15-hydroxystearate) was determined to provide a reference for determining free drug. 40 mg of Plinabulin raw material was added to 6.0 g of propylene glycol, then added to 200 ml of D5W. The resulting solution was continuously shaken at 25 °C and 100 rpm for 48 h. The test solution was centrifuged and filtered through a 0.45 μm nylon filter. The supernatant and subsequent filtrate were injected into the HPLC system, and peak areas were recorded, as shown in Table 2-5.

Table 2-5 Solution Processing Study-Centrifugation and Nylon Filtration

| Name | Peak Area |
|---|---|
| Centrifugation-supernatant | 7122 |
| Centrifugation supernatant-acetonitrile (1:1) | 25537 |
| Nylon-continuous filtrate | No peak value |
| Nylon-continuous filtrate-acetonitrile (1:1) | 1121 |

The test data showed that the 0.45 μm nylon filter membrane had significant adsorption of Plinabulin; dilution of the centrifuged supernatant with acetonitrile resulted in a significant increase in the peak area of Plinabulin, indicating that the supernatant still contained undissolved Plinabulin. Therefore, for Plinabulin, direct centrifugation method cannot be used to prepare the test solution, and suitable filter membranes should be selected for filtration.

After shaking for 52h, the test solution was filtered through a 0.45 μm glass fiber membrane and diluted. The subsequent filtrate was mixed with acetonitrile to verify the dissolution state of Plinabulin in the filtrate. The corresponding continuous filtrate was injected into the HPLC system, and peak areas were recorded, as shown in Table 2-6.

Table 2-6 Solution Processing Study-Glass Fiber Filtration

| Name | Peak Area | Percentage % |
|---|---|---|
| Glass fiber continuous filtrate-0min | 9105 | 100% |
| Glass fiber continuous filtrate-20min | 8936 | 98.1% |
| Glass fiber continuous filtrate-60min | 8866 | 97.4% |
| Glass fiber continuous filtrate-80min | 8872 | 97.4% |
| Glass fiber continuous filtrate-acetonitrile (1:1)-0min | 5691 | 100% |
| Glass fiber continuous filtrate-acetonitrile (1:1)-60min | 5589 | 98.2% |
| Glass fiber continuous filtrate-acetonitrile (1:1)-80min | 5618 | 98.7% |

The test results showed that for the solution of Plinabulin raw material directly filtered through a 0.45μm glass fiber membrane, the peak area after filtration was the peak area of the acetonitrile-diluted filtrate multiplied by the dilution factor.

**Equilibrium Solubility**

Corresponding propylene glycol-DSW injections were prepared. The preparation process was as follows:

Diluent 1 (corresponding to a dilution ratio of 1:20). Weigh about 6g of propylene glycol, place it in a beaker, add 200ml of D5W injection, and mix well.
Diluent 2 (corresponding to a dilution ratio of 1:30). Weigh about 4g of propylene glycol, place it in a beaker, add 200ml of D5W injection, and mix well.
Diluent 3 (corresponding to a dilution ratio of 1:50): Weigh about 2.4g of propylene glycol, place it in a beaker, add 200ml of D5W injection, and mix well.

The protocol for the equilibrium solubility study is shown in Table 2-7.

Table 2-7 Equilibrium Solubility Study Protocol

| **Item** | **Content** |
|---|---|
| Column | ACE C18 (4.6mm×150mm, 5.0μm) |
| Mobile phase | A: 0.01mol/L phosphate buffer (take 0.82g±0.01 of sodium dihydrogen phosphate monohydrate and 1.20g±0.01g of sodium phosphate, dissolve in water and dilute to 1000ml, mix well.);<br>B: Acetonitrile<br>Gradient elution |
| Other chromatographic parameters | Flow rate: 1.2ml/min, Column temperature: 40°C; Injector temperature: 30°C; Detection wavelength: 330nm; Injection volume: 10μl |
| Control solution | 0.2mg/ml Plinabulin solution in absolute ethanol |
| Test solution | Take 20mg of Plinabulin raw material, weigh 3 portions in parallel, add 80ml of the above diluents respectively, shake in a thermostatic water bath oscillator (25°C, 100 rpm) for 48, 72, 96 hours, take an appropriate amount of solution at each time point and filter through a glass fiber membrane (0.45μm), collect the continuous filtrate. |

Acceptance criteria: The concentration of Plinabulin in each solution was determined until there was no significant difference between two consecutive determinations, i.e., equilibrium solubility.

In the content determination method of this product, the reference solution was prepared with absolute ethanol. Since this product was used to determine plasma protein in D5W solution, the influence of different diluents on the determination was investigated. The experimental design was as follows, see Table 2-8.

Table 2-8 Confirmation of Control Diluent

| **Control Solution** | **Preparation Process** |
|---|---|
| Diluted with Absolute Ethanol | Accurately measure an appropriate amount of control stock solution, and dilute with absolute ethanol to prepare a solution containing about 0.1μg of Plinabulin per 1ml. |
| Diluents 1, 2 and 3 | Accurately measure an appropriate amount of control stock solution, and dilute with the above three diluents to prepare a solution containing about 0.1μg of Plinabulin per 1ml. |

10μl of control solutions prepared with different diluents and 48-hour test solutions were injected into the high-performance liquid chromatography system, and the solubility of Plinabulin in the solution was calculated by the external standard method. The results were shown in Table 2-9.

Table 2-9 Different Control Diluents - Solubility Results

| Name | Control Group - Absolute Ethanol Test Concentration (μg/ml) | Control Group - Diluent Test Concentration (μg/ml) |
|---|---|---|
| Test Sample 1 (49.5h) | 0.152 | 0.155 |
| Test Sample 2 (48.5h) | 0.125 | 0.130 |
| Test Sample 3 (49h) | 0.113 | 0.117 |

The results showed that there was no significant difference in the determination results of Plinabulin control solutions prepared with anhydrous ethanol solution and diluent (propylene glycol/D5W injection) in various diluents. Therefore, in the subsequent encapsulation rate determination method, Plinabulin control solution was prepared with absolute ethanol.

Test solutions were taken at different time points, filtered through a 0.45μm glass fiber membrane, and injected into the high-performance liquid chromatography system. The equilibrium solubility of Plinabulin in solutions with different ratios at various time points was calculated by the external standard method. The results were shown in Table 2-10.

Table 2-10 Equilibrium Solubility of Plinabulin at Different Dilution Levels

| Test Sample | Time | Test Concentration (μg/ml) |
|---|---|---|
| Test Sample 1 (1:20) dilution level | 49.5h | 0.152 |
| | 72h | 0.145 |
| | 94h | 0.143 |
| Test Sample 2 (1:30) dilution level | 48.5h | 0.125 |
| | 73h | 0.112 |
| | 94.5h | 0.113 |
| Test Sample 3 (1:50) dilution level | 49h | 0.113 |
| | 73.5h | 0.095 |
| | 96h | 0.134 |
| | 120h | 0.135 |

**Encapsulation Rate of Diluted Injectable Plinabulin**

Based on the above research, the final method for determining encapsulation efficiency was as follows.

(1:10) and (1:20) diluted test solutions: Take Plinabulin concentrate (4mg/ml Plinabulin in propylene glycol/polyoxyethylene 15 hydroxystearate, 60:40 (weight ratio)), place in a 250ml volumetric flask, add 100ml D5W injection (corresponding to 1:10 dilution) or 200ml one (corresponding to 1:20 dilution), shake up and down 30 times, 1 minute.

(1:30) and (1:50) diluted test solutions: Take injectable Plinabulin concentrate (4mg/ml Plinabulin in propylene glycol/polyoxyethylene 15 hydroxystearate, 60:40 (weight ratio)), place in a 500ml volumetric flask, add 300ml D5W injection (corresponding to 1:30 dilution) or 500ml one (corresponding to 1:50 dilution), shake up and down 30 times, 1 minute.

Free test solution: Take about 4ml of the test solution at each dilution level, place in the inner tube of an ultrafiltration centrifuge tube with a molecular weight of 30kd regenerated cellulose, centrifuge at 4500g, discard the ultrafiltration centrifuge tube every 10 minutes. Add about 4ml of test solution to all solutions, continue centrifuging, repeat the operation 6 times (centrifuge for a total of 60 minutes), and take the 60-minute filtrate as the free test solution.

Preparation of total test solution: Take an appropriate amount of each diluted test solution, dilute with absolute ethanol to prepare a solution containing about 2.5μg of Plinabulin per 1ml, as the total test solution.

Control stock solution: Weigh accurately 25mg of Plinabulin reference standard, place in a 100ml volumetric flask, dissolve with absolute ethanol and dilute to the mark, mix well.

Preparation of control solution: Accurately measure 1ml of the above control stock solution, place in a 100ml volumetric flask, dilute to the mark with absolute ethanol, mix well, as the control solution.

Sensitivity solution: Accurately measure an appropriate amount of the above control solution, dilute with absolute ethanol to prepare a solution containing about 0.05μg of Plinabulin per 1ml.

Chromatographic conditions: Octadecylsilane bonded silica gel was used as the filler (ACE C18

4.6mm×150mm, 5.0 μm or equivalent column); 0.01mol/L phosphate buffer (dissolve 0.82g±0.01g of sodium dihydrogen phosphate monohydrate and 1.20g±0.01g of sodium phosphate in water and dilute to 1000ml, mix well) was used as mobile phase A, and acetonitrile was used as mobile phase B. The gradient elution was performed according to the following table; the flow rate was 1.2 ml per minute; the column temperature was 40°C; the injector temperature was 30°C; the detection wavelength was 330 nm; and the injection volume was 20 μl (see Table 2-11).

Table 2-11

| Time (minutes) | Mobile Phase A (%) | Mobile Phase B (%) |
|---|---|---|
| 0 | 90 | 10 |
| 4.0 | 60 | 40 |
| 15.0 | 55 | 45 |
| 16.0 | 20 | 80 |
| 17.0 | 20 | 80 |
| 17.1 | 90 | 10 |
| 20.0 | 90 | 10 |

System suitability requirements: For 5 consecutive injections of the control solution, the relative deviation of the Plinabulin peak area should not exceed 2.0%; in the chromatogram of the sensitivity solution, the signal-to-noise ratio of the Plinabulin peak height should be greater than 10.

Determination method: Accurately measure the control solution, total test solution, and free test solution, inject into the liquid chromatography system, record the chromatograms, and calculate the peak areas by the external standard method. The calculation method is as follows:

$$C_{free} = \frac{A_{free} \times C_{control}}{A_{control}}$$

$$C_{total} = \frac{A_{total} \times C_{control} \times f}{A_{control}}$$

$$\text{Encapsulation Rate (\%)} = \frac{(C_{total} \times C_{free})}{C_{total}} \times 100\%$$

$C_{control}$: control concentration (μg/ml).
$A_{control}$: control peak area.
$C_{free}$: concentration of free test sample (μg/ml).
$A_{free}$: peak area of free test sample.
$C_{total}$: concentration of total test sample (μg/ml).
$A_{total}$: peak area of total test sample.
f: dilution factor of total test sample.

Using the above method, the encapsulation rates of each dilution level for 6 batches of samples were measured, and the measurement results were as follows.

Table 2-12 Encapsulation Rate Results for Each Dilution Level of Each Batch

| Batch No. | **Dilution Level** | **Encapsulation Rate (%)** |
|---|---|---|
| 4A | (1:10) | 99.09 |
| | (1:20) | 97.52 |
| | (1:30) | 96.12 |
| | (1:50) | 96.62 |

(continued)

| Batch No. | **Dilution Level** | **Encapsulation Rate (%)** |
|---|---|---|
| 5A | (1:10) | 99.04 |
| | (1:20) | 97.09 |
| | (1:30) | 98.31 |
| | (1:50) | 95.44 |
| 7A | (1:10) | 99.05 |
| | (1:20) | 98.84 |
| | (1:30) | 98.43 |
| | (1:50) | 94.65 |
| 14A | (1:10) | 98.03 |
| | (1:20) | 98.29 |
| | (1:30) | 98.06 |
| | (1:50) | 96.00 |
| 6A | (1:10) | 98.89 |
| | (1:20) | 98.12 |
| | (1:30) | 97.88 |
| | (1:50) | 97.55 |
| 17A | (1:10) | 99.05 |
| | (1:20) | 98.51 |
| | (1:30) | 98.39 |
| | (1:50) | 97.46 |

The results showed that as the dilution ratio increased, the encapsulation rate of Plinabulin micelles decreased.

**Effect of Stirring Method on Encapsulation Rate**

The effects of different shaking times and different shaking methods on micelle characteristics were studied. The samples from batch 004A were used, concentrated Plinabulin (4mg/ml, in propylene glycol/poly(ethylene glycol) 15-hydroxystearate) was diluted with D5W at dilution ratios of 1:10, 1:20, 1:30, and 1:50. The mixtures were prepared using manual shaking at different frequencies or vortexing for different durations, and the encapsulation rates were determined. The stirring methods were as follows.

Table 2-13 Shaking Method Study - Specific Parameters

| **Manual shaking** | vortexing |
|---|---|
| 30 times for 1 minute | 1 minute |
| 90 times for 1 minute | 3 minutes |

The encapsulation rate results were as follows.

Table 2-14 Shaking Method Study - Encapsulation Rate Results for Different Dilution Levels

| Dilution Level | Shaking Method | Encapsulation Rate (%) |
|---|---|---|
| (1:10) | Manual shaking 30 times for 1 minute | 98.03 |
| | Manual shaking 90 times for 1 minute | 99.09 |
| | Vortexing for 1 minute | 99.09 |
| | Vortexing for 3 minutes | 99.12 |
| (1:20) | Manual shaking 30 times for 1 minute | 98.29 |
| | Manual shaking 90 times for 1 minute | 98.16 |
| | Vortexing for 1 minute | 98.83 |
| | Vortexing for 3 minutes | 98.70 |
| (1:30) | Manual shaking 30 times for 1 minute | 98.06 |
| | Manual shaking 90 times for 1 minute | 98.35 |
| | Vortexing for 1 minute | 98.24 |
| | Vortexing for 3 minutes | 97.48 |
| (1:50) | Manual shaking 30 times for 1 minute | 96.00 |
| | Manual shaking 90 times for 1 minute | 95.72 |
| | Vortexing for 1 minute | 97.50 |
| | Vortexing for 3 minutes | 97.36 |

The results showed that for samples prepared using the four methods - manual shaking 30 times for 1 minute, manual shaking 90 times for 1 minute, vortexing for 1 minute, and vortexing for 3 minutes - the encapsulation rate of each sample was between 95% and 99%. Within this range, it indicated that diluting samples using different shaking methods had little effect on the encapsulation rate. Therefore, it can be inferred that the encapsulation rate of sample solutions diluted by different personnel remains relatively stable in clinical use.

**Dilution Resistance Study of Diluted Formulation in PBS Solution**

Batch samples (dilution ratios of 1:10, 1:20, 1:30, and 1:50) were diluted and used to simulate the clinical infusion process. The diluted test solution was controlled to be infused into 5L of pH 7.4 phosphate buffer solution within about 30 minutes. Based on the human blood volume (about 7%-8% of body weight, for a 60kg weight, blood volume is about 4.2L-4.8L), 5L of pH 7.4 phosphate buffer solution was used in this study to simulate human blood volume. Sampling points were at 10 minutes, 20 minutes, and completion of infusion (except for the test solution with a dilution ratio of 1:50, where the infusion completion time was about 60 minutes, with sampling points at 20 minutes, 40 minutes, and completion of infusion).

Due to the low drug concentration in the dilution resistance test (total concentration in the range of 7μg/ml-8μg/ml) and the phosphate buffer system, it was determined that regenerated cellulose filter membranes with a molecular weight cut-off of 30kd had filtration adsorption effects at this concentration level.

(1:20) dilution level solution: Accurately weigh about 10.36g of plasma concentrate, place in a 250ml volumetric flask, add 200ml of D5W injection, shake up and down 30 times within 1 minute to obtain the solution.

Free test solution: Take 2ml of the above diluted solution, place in 50ml of pH 7.4 phosphate buffer solution (37°C), and stir for 1 minute. Take about 4ml of solution (prepare new solution every 10 minutes and replace the solution in the centrifuge tube), place in the inner tube of an ultrafiltration centrifuge tube (regenerated cellulose, molecular weight 30kd), centrifuge (speed 4500g) for 20min, 40min, 50min, 60min, 70min, 80min, and take the filtrate as the free test solution.

Preparation of total test solution: Accurately measure 2ml of the above solution, place in a 50ml volumetric flask, dilute to the mark with absolute ethanol, mix well, filter, and use as the total test solution.

The free test solution and the total test solution were taken and injected into the liquid chromatography system. The concentration of Plinabulin in the filtrate at different centrifugation times was calculated using the external standard method, and the adsorption situation of the filter membrane was determined. The results were as follows.

Table 2-15 Filter Membrane Adsorption Study - Regenerated Cellulose Filter Membrane

| Sample | Concentration (μg/ml) | Encapsulation Rate (%) |
|---|---|---|
| Total | 7.7088 | / |
| Membrane 30kd(1-20)-20min | 1.0239 | 86.72 |
| Membrane 30kd(1-20)-40min | 1.3037 | 83.09 |
| Membrane 30kd(1-20)-60min | 1.3084 | 83.03 |
| Membrane 30kd(1-20)-70min | 1.3509 | 82.48 |
| Membrane 30kd(1-20)-80min | 1.3173 | 82.91 |

Through the adsorption study of the dilution-resistant filter membrane, it can be seen that the regenerated cellulose filter membrane with a molecular weight cut-off of 30kd can reach adsorption saturation after 40 minutes.

To simulate the clinical use process, the solutions at different dilution levels (1: 10, 1:20, 1:30, 1:50) were prepared, and the diluted solutions were infused into 5L of pH 7.4 phosphate buffer solution within about 30 minutes (note: the infusion time for the 1:50 dilution level solution was about 1 hour), to study the changes in encapsulation rate during the infusion process. The results were as follows.

Table 2-16 Encapsulation Rate Results for Different Dilution Levels

| Dilution Level | Sample | Encapsulation Rate (%) |
|---|---|---|
| (1:10) | Infused 10 min (infused about 1/3 of the volume) | 21.77 |
| | Infused 20 min (infused about 2/3 of the volume) | 67.82 |
| | Infused 30 min (infusion completed) | 80.34 |
| (1:20) | Infused 10 min (infused about 1/3 of the volume) | 64.30 |
| | Infused 20 min (infused about 2/3 of the volume) | 78.65 |
| | Infused 30 min (infusion completed) | 82.44 |
| (1:30) | Infused 10 min (infused about 1/3 of the volume) | 52.83 |
| | Infused 20 min (infused about 2/3 of the volume) | 76.71 |
| | Infused 30 min (infusion completed) | 82.13 |
| (1:50) | Infused 20 min (infused about 1/3 of the volume) | 63.45 |
| | Infused 40 min (infused about 2/3 of the volume) | 70.57 |
| | Infused 60 min (infusion completed) | 80.41 |

Based on the concentration of the total test solution during the infusion process, the actual infusion amount of the test sample and the concentration of poly(ethylene glycol) 15-hydroxystearate at the corresponding time points were calculated. The results were as follows.

Table 2-17 Analysis of Infusion Results at Different Dilution Levels

| Dilution Level | Sample | Total Test Concentration (μg/ml) | Actual Sample Infusion Volume (%) | Concentration of poly(ethylene glycol) 15-hydroxystearate (%) |
|---|---|---|---|---|
| (1:10) | Infused 10 min (infused about 1/3 of the volume) | 1.1987 | 17.6 | 0.014% |
| | Infused 20 min (infused about 2/3 of the volume) | 3.9167 | 57.6 | 0.047% |
| | Infused 30 min (infusion completed) | 6.8005 | 100.0 | 0.081% |

(continued)

| Dilution Level | Sample | Total Test Concentration (μg/ml) | Actual Sample Infusion Volume (%) | Concentration of poly(ethylene glycol) 15-hydroxystearate (%) |
|---|---|---|---|---|
| (1:20) | Infused 10 min (infused about 1/3 of the volume) | 2.9091 | 39.9 | 0.032% |
| | Infused 20 min (infused about 2/3 of the volume) | 5.5796 | 76.5 | 0.061% |
| | Infused 30 min (infusion completed) | 7.2965 | 100.0 | 0.079% |
| (1:30) | Infused 10 min (infused about 1/3 of the volume) | 2.5144 | 34.8 | 0.028% |
| | Infused 20 min (infused about 2/3 of the volume) | 4.8905 | 67.7 | 0.054% |
| | Infused 30 min (infusion completed) | 7.2274 | 100.0 | 0.078% |
| (1:50) | Infused 20 min (infused about 1/3 of the volume) | 2.5629 | 37.0 | 0.029% |
| | Infused 40 min (infused about 2/3 of the volume) | 5.5209 | 79.7 | 0.061% |
| | Infused 60 min (infusion completed) | 6.9243 | 100.0 | 0.075% |

The results showed that the encapsulation rate for the first 1/3 volume of infusion was relatively low, with significant variations. The critical micelle concentration (CMC) of poly(ethylene glycol) 15-hydroxystearate in the poly(ethylene glycol) 15-hydroxystearate excipient are between 0.005% and 0.02%. As a reference, the critical micelle concentration of poly(ethylene glycol) 15-hydroxystearate determined by steady-state fluorescence method was 0.0035%. Combining the above infusion process and data, it can be seen that after 10 minutes of infusion at the (1:10) dilution level, the actual infusion amount was only 17.6%, and the concentration of poly(ethylene glycol) 15-hydroxystearate in PBS solution was at the critical micelle concentration level, resulting in a lower encapsulation rate. When the solutions at other dilution levels reached 1/3 of the pre-infusion amount, the concentration of poly(ethylene glycol) 15-hydroxystearate in PBS solution was slightly higher than the critical micelle concentration and that for the (1:10) dilution level, with an encapsulation rate slightly higher than 50% to 60%.

As the infusion time increased, the concentration of poly(ethylene glycol) 15-hydroxystearate gradually increased, and the encapsulation efficiency also gradually improved. In 5L pH 7.4 phosphate buffer solution, the final encapsulation rates of test solutions at different dilution levels all exceeded 80%.

The encapsulation rate results showed that when solutions at various dilution levels reached 1/3 of the pre-infusion amount, the amount of poly(ethylene glycol) 15-hydroxystearate in PBS solution reached or slightly exceeded the critical micelle concentration. The encapsulation rate results were relatively low. As the infusion time extended, the diluted solution gradually formed micelles in the buffer salt, and the encapsulation rate gradually increased to a level of 80%.

**Micelle Stability Study of Diluted Formulations**

Batch samples of Plinabulin were diluted at ratios of 1:10, 1:20, 1:30, and 1:50. The diluted solutions were stored at room temperature protected from light, and samples were taken at 0h, 2h, 4h, 6h, 8h, 12h, and 24h to study the stability of micelle properties. The results were as follows.

Table 2-18 Encapsulation Rate Results for Different Dilution Levels

| Dilution Level | Time | Total Test Sample | | Encapsulation Rate (%) |
|---|---|---|---|---|
| | | Concentration (μg/ml) | Percentage (%) | |
| (1:10) | 0h | 348.1729 | 100.00 | 99.14 |
| | 2h | 347.1970 | 99.72 | 99.15 |
| | 4h | 351.5851 | 100.98 | 99.10 |
| | 6h | 346.7893 | 99.60 | 98.01 |
| | 8h | 350.4087 | 100.64 | 97.33 |
| | 12h | 346.1209 | 99.41 | 99.07 |
| | 24h | 343.4498 | 98.64 | 99.15 |
| (1:20) | 0h | 182.8625 | 100.00 | 98.70 |
| | 2h | 181.8081 | 99.42 | 98.65 |
| | 4h | 183.6379 | 100.42 | 98.28 |
| | 6h | 182.7489 | 99.94 | 98.76 |
| | 8h | 181.9084 | 99.48 | 98.75 |
| | 12h | 187.6015 | 102.59 | 98.68 |
| | 24h | 184.5427 | 100.92 | 98.75 |
| (1:30) | 0h | 126.8501 | 100.00 | 98.41 |
| | 2h | 126.8258 | 99.98 | 97.62 |
| | 4h | 126.5170 | 99.74 | 98.33 |
| | 6h | 126.0722 | 99.39 | 98.38 |
| | 8h | 126.7503 | 99.92 | 98.22 |
| | 12h | 128.2439 | 101.10 | 98.41 |
| | 24h | 128.1423 | 101.02 | 98.42 |
| (1:50) | 0h | 79.4270 | 100.00 | 97.47 |
| | 2h | 77.2850 | 97.30 | 97.55 |
| | 4h | 77.4346 | 97.49 | 97.50 |
| | 6h | 77.2085 | 97.21 | 97.49 |
| | 8h | 76.7789 | 96.67 | 97.60 |
| | 12h | 78.4067 | 98.72 | 97.58 |
| | 24h | 76.4981 | 96.31 | 97.68 |

The results indicated that when the prepared test solutions at various dilution levels were stored at room temperature protected from light for 24 hours, the concentration of the total test solution at 0 hour was calculated as 100.0%, and the concentrations of the total test solution at various time points ranged from 96.0% to 103.0%; the encapsulation rate remained above 95%, indicating good stability of the micelle properties in the diluted solutions.

This product was diluted with D5W injection at different ratios (1:10, 1:20, 1:30, 1:50) and the total test solution was stored at room temperature protected from light for 24 hours. There were no significant changes in concentration and encapsulation rate, indicating that the micelle solution was stable at room temperature for 24 hours.

**Determination of Critical Micelle Concentration**

Critical micelle concentration (CMC) is used to evaluate the micellar characteristics of substances. Common measurement methods include surface tension method, conductivity method, fluorescence probe method, dye method, etc. Among these, the fluorescence probe method has been widely used in determining the critical micelle concentration of

surfactants due to its advantages such as simple operation and minimal interference with the research system. Therefore, it was decided to use the fluorescence probe method to measure the concentration of poly(ethylene glycol) 15-hydroxystearate in the formulation system and critical microsphere concentrations in 5% dextrose injection and pH 7.4 PBS solution, respectively. The equipment and reagents used in the study were summarized as follows.

Pyrene Stock Solution

Weigh 20mg of pyrene as a fluorescent reagent, place it in a 10ml volumetric flask, dissolve with acetone and dilute to the mark, mix well. The concentration of pyrene in the solution was 2.0mg/ml ($1.0\times10^{-5}$ mol/ml).

Propylene glycol-poly(ethylene glycol) 15-hydroxystearate Stock Solution

Propylene glycol: poly(ethylene glycol) 15-hydroxystearate = 60:40 (wt:wt). Weigh about 7.5g of propylene glycol and about 5g of poly(ethylene glycol) 15-hydroxystearate, place in a 50ml volumetric flask, dissolve with acetone and dilute to the mark, mix well, with a concentration of 100mg/ml.

Sample Preparation

0.1ml of pyrene stock solution was taken and placed into ten 10 ml volumetric flasks. After the acetone was evaporated to dryness, different amounts of propylene glycol-poly(ethylene glycol) 15-hydroxystearate stock solution were added. After the acetone was evaporated to dryness, the mixture was diluted with D5W injection to the mark and mixed well. The concentrations of poly(ethylene glycol) 15-hydroxystearate micelles in the solutions are $1\times10^{-7}$ g/ml, $5\times10^{-7}$g/ml, $1\times10^{-6}$g/ml, $5\times10^{-6}$g/ml, $1\times10^{-5}$g/ml, $5\times10^{-5}$g/ml, $1\times10^{-4}$ g/ml, $5\times10^{-4}$g/ml, $1\times10^{-3}$g/ml, $1\times10^{-2}$g/ml. Measurements were taken after 24 hours at room temperature.

A series of solutions with identical concentrations were prepared in parallel, diluted to the mark with pH 7.4 PBS solution, and measured after 24 hours at room temperature.

The specific measurement parameters of the fluorescence spectrophotometer were as follows.

Table 2-19 Fluorescence Spectrophotometer Setting Parameters

| **Item** | **Specific Parameters** |
|---|---|
| Excitation wavelength | 336nm |
| Emission wavelength range | 350nm~450nm |
| Excitation slit | 5.0nm |
| Emission slit | 2.5nm |
| Scanning speed | 1200nm/min |
| Voltage | 700V |
| Test number | 3 times per sample |

The fluorescence spectra of the two groups of solutions were measured after 24 hours at room temperature according to the above method.

The CMC values of poly(ethylene glycol) 15-hydroxystearate in different solution systems were obtained by curve fitting using the Boltzmann formula.

Table 2-20

| Solution System | poly(ethylene glycol) 15-hydroxystearate-CMC | |
|---|---|---|
| | Concentration | Concentration |
| D5W solution | $4.43\times10^{-5}$g/ml | 0.0044% |
| PBS solution | $3.14\times10^{-5}$g/ml | 0.0031% |

In the excipient specifications, the CMC was in the range of 0.005% to 0.02%. The CMC of poly(ethylene glycol) 15-hydroxystearate in water was determined to be 0.0035%, which served as a control. There was no significant difference in the CMC of poly(ethylene glycol) 15-hydroxystearate in different solution systems.

**Example 3**

This example describes the results of a dilution study conducted to determine the appearance, assay, impurities, and microbial evaluation of Plinabulin (4mg/mL) (propylene glycol/poly(ethylene glycol) 15-hydroxystearate solution) at different time points after dilution in 5% dextrose (D5W) in non-PVC intravenous infusion bags (500mL). In this example, 4mg/mL Plinabulin samples from six (6) clear vials and six (6) turbid vials were diluted with D5W to obtain two dilution levels: about 1:20 and about 1:200.

The Plinabulin (4mg/mL) vials became turbid when refrigerated and were then heated at 37°C for 1 hour before dilution to reverse the turbidity. The diluted samples were initially (0 hour) tested at room temperature and tested again after storage protected from light for 4 hours, 6 hours, 8 hours, 12 hours, 24 hours, and 48 hours. The post-dilution stability and potential microbial risk of Plinabulin (4mg/mL) were determined at 6 hours, 8 hours, 12 hours, 24 hours, and 48 hours after dilution with D5W in non-PVC intravenous infusion bags (500ml).

Two batches of Plinabulin (4mg/mL) were used in this study. The first batch was used for chemical and appearance analysis, and the second batch was used for microbial count testing.

A filter interference study was conducted on the 1:20 and 1:200 dilutions using the first batch of product. For the filter interference study, the 1:200 dilution did not meet the acceptable criteria, so unfiltered samples were used throughout the study.

For the dilution study and microbial count study, the results for clear vials and vials that became turbid due to refrigeration were the same. All samples at both dilution levels and all time points met the acceptable criteria for microbial count.

The samples diluted at 1:20 met the stability criteria for assay at 0, 4, 6, 8, and 12 hours. The samples diluted at 1:20 failed to meet the acceptable criteria at 24 hours and 48 hours after dilution due to the presence of precipitate and potency less than 90% of the label requirements. The samples diluted at 1:200 met the stability criteria for assay within 0, 4, 6, and 8 hours after dilution. The samples diluted at 1:20 failed to meet the acceptable criteria at 12, 24, and 48 hours after dilution due to the presence of precipitate and potency less than 90% of the label requirements.

No impurities ≥0.10% were detected in clear and turbid vials at the 1:20 dilution level. For the 1:200 dilution level, the impurities present were below the detection level and were not quantified.

**Filter Interference Study**

The potential interference of 0.2 micron PBS filters was evaluated by filtering and analyzing a portion of the samples. A portion of filtered samples drawn from the port with an infusion tube was compared with a portion of unfiltered samples drawn directly from the outlet port. Three (3) samples were prepared and tested at each dilution level (1:20 and 1:200). A placebo control sample was prepared at the 1:20 dilution level to identify placebo peak value. For the 1:20 dilution level, the assay values and impurity values obtained from the filtered portion were compared with those from the unfiltered portion. For the 1:200 dilution, the assay values obtained from the filtered portion were compared with those from the unfiltered portion. The results of the filter interference study were listed in Table 3-1.

Dilution Study Method

In this study, 4mg/mL Plinabulin injection was diluted with D5W in non-PVC intravenous infusion bags to generate diluted intravenous infusions.

The volume of D5W solution contained in individual non-PVC intravenous infusion bags (500mL) was measured to full volume by pouring the solution in the bag into a 500mL graduated cylinder or volumetric flask and using an appropriately sized graduated cylinder with an accuracy of 1mL or better. The average volume (n=4) was determined for use in sample calculations.

All D5W non-PVC N bags used for determining the average volume were from the same manufacturer/supplier and the same manufacturer's batch as all bags used for the dilution study.

The required volume of Plinabulin (4mg/mL) was determined according to Formula 2. A nominal concentration of 0.2 mg/mL was obtained at a dilution of 1:20. For example, 12.5 mL (±0.2 mL) was aseptically withdrawn from each of two (2) vials of Plinabulin (4 mg/mL) using a graduated syringe and injected directly into a 500 mL non-PVC intravenous injection bag covered with an amber colored sleeve via a single injection. The weight of Plinabulin (4 mg/mL) injected into the bag was recorded. A nominal concentration of 0.02 mg/mL was obtained at a dilution of 1:200. For example, 2.5 mL (±0.05 mL) was aseptically withdrawn from a vial containing Plinabulin (4 mg/mL) using a graduated syringe and injected directly into a 500 mL non-PVC intravenous bag covered with an amber colored sleeve by a single injection. The weight of Plinabulin (4 mg/mL) injected into the bag was recorded. Note: Impurities present below detection level were not quantified at a dilution of 1:200.

The infusion bag was gently rotated to thoroughly mix the infusion solution. The appearance of the diluted

solution in the non-PVC infusion bag was checked and recorded. The diluted solution was stored in the non-PVC infusion bag and kept at controlled room temperature (25°C ± 3°C) protected from light for a total of 48 hours. The actual bag volume after injection was calculated using Formula 3 and the label requirements after each dilution were calculated using Formula 4. The appearance of the diluted solution at each time interval was inspected and recorded.

Samples were taken directly from the intravenous injection bag and not from the intravenous tubing. Chemical assay and impurity testing were performed as described herein. Using the same intravenous tubing, samples were withdrawn directly through the tubing (without priming) and recorded at each time interval. The first batch was used for chemical testing. Microbial testing was performed using USP <61>. Samples were drawn through the outlet port and recorded at each interval. The results of the dilution studies were listed in Table 25. The second batch was used for microbial enumeration studies.

Formula 2

$$\text{Required volume}_{\text{Plinabulin injection (4 mg/ml)}} = (C_{\text{IV bag}}\text{, mg/ml} * V_{\text{IV bag}}\text{, ml})/(4\text{ mg/ml}).$$

Where $C_{\text{IV bag}}$ is the desired final concentration of Plinabulin in the diluted intravenous injection bag in mg/ml (i.e.: 0.2mg/ml or 0.02mg/ml), $V_{\text{IV bag}}$ is the expected initial volume of D5W in the intravenous injection bag in ml from the manufacturer's product description.

Formula 3

Actual $\text{volume}_{\text{IV bag}}$, ml = $V_{\text{average}}$, ml + (($\text{Wt.}_{\text{Plinabulin injection (4 mg/ml)}}$, g)/($d_{\text{Plinabulin injection (4 mg/ml)}}$, g/ml)). Where $V_{\text{average}}$ refers to the average volume measured from 4 intravenous injection bags in ml. $\text{Wt.}_{\text{Plinabulin injection (4 mg/ml)}}$ is the actual weight of Plinabulin injection (4 mg/ml) added to the intravenous injection bag in g, $d_{\text{Plinabulin injection (4 mg/ml)}}$ is the density of Plinabulin injection (4 mg/ml) obtained from the executed batch record.

Formula 4

Label $\text{requirement}_{\text{IV solution}}$, mg/ml = (4 mg/ml * $\text{Wt}_{\text{Plinabulin injection (4 mg/ml)}}$, g)/(($d_{\text{IV solution (4 mg/ml)}}$, g/ml * $V_{\text{IV bag}}$, ml). Where $\text{Wt}_{\text{Plinabulin injection (4 mg/ml)}}$ refers to the actual weight added to the intravenous injection bag in g, $d_{\text{Plinabulin injection (4 mg/ml)}}$ refers to the density of Plinabulin injection (4 mg/ml) obtained from the executed batch record, $V_{\text{IV bag}}$ refers to the actual volume in the intravenous injection bag as determined by Formula 2.

## Results

Filter Interference Study

The assay results for the filter interference study at the 1:20 dilution level met the acceptable criteria of within 3.0%. The assay results at the 1:200 dilution level did not meet the acceptable criteria of within 3.0%. Since filter interference was observed in the assay at the 1:200 dilution level, unfiltered samples were used in the study. No impurities (~0.10%) were detected in both filtered and unfiltered samples. Testing was performed using the first batch of samples.

Table 3-1 Filter Interference Study

| Performance Characteristic | Evaluation Parameter | Acceptance Criteria | Results (% Diff.) |
|---|---|---|---|
| Assay (1:20) | % Difference between filtered portion and unfiltered portion | Within 3.0% of unfiltered sample solution | 0.3, 0.1, 1.4 |
| Assay (1:200) | | | 5.6, 6.5, 4.8 |
| Impurities (1:20) | | For individual reported impurities ≤ 0.5%, the absolute difference is 0.1% NMT of unfiltered impurities | No impurities ≥ 0.10% were detected for filtered and unfiltered samples |

## Dilution Study

Results of the dilution study were listed in Tables 3-2, 3-3, and 3-4. Testing was performed using batch 2. Microbial testing was performed using batch 1. Samples were withdrawn from the intravenous injection bags, infusion

tubes, or outlet ports (if applicable).

Table 3-2 Dilution Study

| **Test** | **Sample Volume** | **Test Method** | **Results** |
|---|---|---|---|
| Initial vial appearance | Vial | Visual observation | The solution in all vials is clear and yellow before dilution, no heat treatment is required |
| Initial appearance after vial heat treatment (if applicable) | Vial | Visual observation | N/A (All vials became clear after heat treatment prior to dilution.) |
| Appearance of sample in bag | Draw 5 ml | Visual observation | **Clear Vial**<br>**0 hour (start)**<br>*1:20 dilution level:* clear yellow solution observed in all six bags<br>*1:200 dilution level:* light yellow clear solution observed in all six bags<br>**4 hours**<br>*1:20 dilution level:* clear yellow solution observed in all six bags<br>*1:200 dilution level:* light yellow clear solution observed in all six bags<br>**6 hours**<br>*1:20 dilution level:* clear yellow solution observed in all six bags<br>*1:200 dilution level:* light yellow clear solution observed in all six bags<br>**8 hours**<br>*1:20 dilution level:* clear yellow solution observed in all six bags<br>*1:200 dilution level:* light yellow clear solution observed in all six bags<br>**12 hours** |
| | | | *1:20 dilution level:* clear yellow solution observed in all six bags<br>*1:200 dilution level:* light yellow clear solution observed in all six bags<br>**24 hours**<br>*1:20 dilution level:* clear yellow solution observed in bags 1-3; clear yellow solution suspended with fine particles observed in bags 4-6<br>*1:200 dilution level:* clear light yellow solution suspended with fine particles observed in in all six bags<br>**48 hours**<br>*1:20 dilution level:* clear yellow solution suspended with fine particles observed in all six bags<br>*1:200 dilution level:* clear light yellow solution suspended with fine particles observed in bags 1-3; clear yellow solution suspended with fine particles observed in bags 4-6 |

(continued)

| Test | Sample Volume | Test Method | Results |
|---|---|---|---|
| Appearance of sample in bag | Draw 5 ml | Visual observation | **Turbid vial**<br>**0 hour (start)**<br>*1:20 dilution level:* clear yellow solution observed in all six bags<br>*1:200 dilution level:* light yellow clear solution observed in all six bags<br>**4 hours**<br>*1:20 dilution level:* clear yellow solution observed in all six bags<br>*1:200 dilution level:* light yellow clear solution observed in all six bags<br>**6 hours**<br>*1:20 dilution level:* clear yellow solution observed in all six bags<br>*1:200 dilution level:* light yellow clear solution observed in all six bags<br>**8 hours**<br>*1:20 dilution level:* clear yellow solution observed in all six bags<br>*1:200 dilution level:* light yellow clear solution observed in all six bags<br>**12 hours**<br>*1:20 dilution level:* clear yellow solution observed in all six bags<br>*1:200 dilution level:* light yellow clear solution observed in all six bags<br>**24 hours**<br>*1:20 dilution level:* clear yellow solution observed in all six bags<br>*1:200 dilution level:* clear light yellow solution suspended with fine particles observed in all six bags<br>**48 hours**<br>*1:20 dilution level:* clear yellow solution suspended with fine particles observed in all six bags<br>*1:200 dilution level:* clear light yellow solution suspended with fine particles observed in all six bags |

Table 3-3 Dilution Study (Continued)

| Test | Sample Volume | Acceptance Criteria | Results / 1:20 dilution level | | | |
|---|---|---|---|---|---|---|
| Assay | 20 ml | 90.0%-110.0% of label requirements | 0 hour (start) | | 4 hours | |
| | | | vial | | vial | |
| | | | clear | turbid | clear | turbid |
| | | | 98.3 | 99.2 | 98.5 | 99.1 |
| | | | 99.1 | 98.7 | 98.9 | 99.1 |
| | | | 98.5 | 98.7 | 102.0 | 99.1 |
| | | | 98.1 | 97.9 | 98.9 | 98.9 |
| | | | 99.1 | 98.9 | 99.2 | 99.0 |
| | | | 99.1 | 98.9 | 99.1 | 99.0 |
| | | | **6 hours** | | **8 hours** | |
| | | | vial | | vial | |
| | | | clear | turbid | clear | turbid |
| | | | 98.9 | 99.3 | 98.6 | 99.3 |
| | | | 98.8 | 99.2 | 99.0 | 99.1 |
| | | | 97.6 | 99.3 | 97.2 | 99.2 |
| | | | 99.0 | 99.0 | 98.9 | 93.0 |
| | | | 99.2 | 99.1 | 99.3 | 99.1 |
| | | | 99.3 | 98.8 | 98.3 | 98.9 |
| | | | **12 hours** | | **24 hours** | |
| | | | vial | | vial | |
| | | | clear | turbid | clear | turbid |
| | | | 97.8 | 99.1 | 98.5 | 84.5 |
| | | | 96.1 | 99.2 | 98.0 | 87.0 |
| | | | 93.7 | 99.1 | 94.7 | 78.2 |
| | | | 98.9 | 99.0 | 85.4 | 72.9 |
| | | | 99.0 | 99.3 | 69.3 | 48.0 |
| | | | 97.9 | 98.9 | 70.8 | 87.1 |
| | | | **48 hours** | | | |
| | | | vial | | | |
| | | | clear | turbid | | |
| | | | 82.0 | 81.8 | | |
| | | | 60.6 | 83.6 | | |
| | | | 35.2 | 91.0 | | |
| | | | 77.4 | 78.6 | | |
| | | | 66.4 | 89.1 | | |
| | | | 61.4 | 62.0 | | |
| Impurities (1:20 dilution level only) | Using assay samples | Impurity Limit<br>Plinabulin - NMT 0.30%<br>Any single unknown impurity ≥ 0.1%<br>Total impurities NMT 1.0% | **0 (start), 4, 6, 8, 12, 24, and 48 hours:**<br>≥ 0.10% impurities undetected in Clear and vials turbid<br>Total impurities: 0.0% | | | |

Table 3-4 Dilution Study (Continued)

| Test | Sample Volume | Acceptance Criteria | Results/1:200 Dilution | | | |
|---|---|---|---|---|---|---|
| Assay | 20 ml | 90.0%-110.0% of label requirements | 0 hour (sta rt) | | **4 hours** | |
| | | | vial | | vial | |
| | | | clear | turbid | clear | turbid |
| | | | 96.3 | 101.1 | 96.3 | 101.4 |
| | | | 99.3 | 101.8 | 99.1 | 101.8 |
| | | | 99.8 | 102.0 | 99.7 | 101.9 |
| | | | 99.9 | 100.9 | 99.9 | 101.0 |
| | | | 99.4 | 101.7 | 99.2 | 101.4 |
| | | | 99.6 | 101.0 | 99.4 | 100.8 |
| | | | **6 hours** | | **8 hours** | |
| | | | vial | | vial | |
| | | | clear | turbid | clear | turbid |
| | | | 96.5 | 101.1 | 96.3 | 101.3 |
| | | | 98.8 | 101.8 | 98.7 | 101.2 |
| | | | 99.6 | 102.2 | 99.3 | 101.6 |
| | | | 100.2 | 101.1 | 100.0 | 100.6 |
| | | | 100.0 | 101.5 | 97.0 | 100.4 |
| | | | 99.6 | 101.1 | 98.6 | 101.0 |
| | | | **12 hours** | | **24 hours** | |
| | | | vial | | vial | |
| | | | clear | turbid | clear | turbid |
| | | | 96.4 | 101.8 | 73.9 | 88.9 |
| | | | 98.2 | 100.9 | 70.3 | 66.2 |
| | | | 97.9 | 99.8 | 49.4 | 65.7 |
| | | | 99.9 | 99.9 | 53.8 | 75.2 |
| | | | 93.7 | 99.3 | 50.5 | 72.9 |
| | | | 89.8 | 101.2 | 50.9 | 64.5 |
| | | | **48 hours** | | | |
| | | | vial | | | |
| | | | clear | | turbid | |
| | | | 61.4 | | 59.3 | |
| | | | 58.2 | | 55.7 | |
| | | | 38.5 | | 49.3 | |
| | | | 39.3 | | 55.5 | |
| | | | 35.9 | | 49.3 | |
| | | | 34.4 | | 44.0 | |

Table 3-5 Microbial Enumeration Study

| Microbial Enumeration Test | Sample Volume | Test Method | Acceptance Criteria | Results |
|---|---|---|---|---|
| 1:20 dilution | 200 ml | Current USP <61> | Total aerobic microbial count: NMT 100 cfu/ml | **6, 8, 12, 24, and 48 hours:** NMT 100 cfu/ml for clear and turbid vials |
| | | | Total molds and yeasts count: NMT 10 cfu/ml | **6, 8, 12, 24, and 48 hours:** NMT 100 cfu/ml for clear and turbid vials |
| 1:200 dilution | | | Total molds and yeasts count: NMT 100 cfu/ml | **6, 8, 12, 24, and 48 hours:** NMT 100 cfu/ml for clear and turbid vials |
| | | | Total molds and yeasts count: NMT 10 cfu/ml | **6, 8, 12, 24, and 48 hours:** NMT 100 cfu/ml for clear and turbid vials |

For the filtration interference study, the 1:200 dilution did not meet acceptable standards, therefore unfiltered samples were used throughout the study.

For the dilution study and microbial count study, the results from clear vials and turbid vials caused by refrigeration were the same. Both dilutions and all samples at all time points met the acceptable standards for microbial counts.

The samples diluted at 1:20 met the stability standards for detection within 0, 4, 6, 8, and 12 hours. The samples diluted at 1:20 did not meet acceptable standards at 24 and 48 hours after dilution due to the presence of precipitates and potency less than 90% of label requirements. The samples diluted at 1:200 met the stability standards for detection within 0, 4, 6, and 8 hours after dilution. The samples diluted at 1:200 failed to meet acceptable standards at 12, 24, and 48 hours after dilution due to the presence of precipitates and potency less than 90% of label requirements.

No impurities ≥0.10% were detected in clear and turbid vials at the 1:20 dilution level. For the 1:200 dilution level, impurities present were below the detection level and were not quantified. Based on the results obtained from clear and turbid vials, it can be concluded that Plinabulin (4 mg/mL) diluted with D5W in non-PVC intravenous injection bags (500 mL) was stable for 12 hours at the 1:20 dilution level and for 8 hours at the 1:200 dilution level when stored at room temperature protected from light.

All literatures mentioned in this invention are incorporated herein by reference as if each individual literature is specifically incorporated by reference. Furthermore, it should be understood that after reading the above teachings of this invention, those skilled in the art can make various modifications or alterations to this invention, and these equivalent forms also fall within the scope defined by the appended claims.

## Claims

**1.** A method for preparing a Plinabulin micelle composition, wherein the method comprises the steps of:

s1) mixing poly(ethylene glycol) 15-hydroxystearate with propylene glycol at 35°C-65°C to prepare a clear mixed solution;
s2) mixing the clear mixed solution from step 1) with Plinabulin at 35°C-65°C and stirring to obtain the Plinabulin micelle composition.

**2.** The method of claim 1, wherein the weight ratio of poly(ethylene glycol) 15-hydroxystearate to propylene glycol is 1:5-5:1, preferably 1:3-3:1, more preferably 2:3.

**3.** The method of claim 1, wherein in step (s2), the Plinabulin is Plinabulin monohydrate.

**4.** A Plinabulin micelle composition, wherein the micelle composition comprises a clear mixed solution of Plinabulin, poly(ethylene glycol) 15-hydroxystearate and propylene glycol, wherein the Plinabulin micelle composition is a yellow clear transparent solution with a micellar particle size range of 10-100 nm.

**5.** The Plinabulin micelle composition of claim 4, wherein the Plinabulin micelle composition is prepared by the method of

claim 1.

**6.** The Plinabulin micelle composition of claim 4, wherein the Plinabulin micelle composition comprises the following components:

propylene glycol 30-90wt%, preferably 40-70wt%, for example 50wt%, 55wt%, 60wt%, 65wt%, 70wt%;
poly(ethylene glycol) 15-hydroxystearate 20-60wt%, preferably 20-50wt%, for example 25wt%, 30wt%, 35wt%, 40wt%, 45wt%; and
Plinabulin 1-10wt%, preferably 2-8wt%, for example 3wt%, 4wt%, 5wt%, 6wt%, 7wt%.

**7.** The Plinabulin micelle composition of claim 4 or 6, wherein the weight ratio of poly(ethylene glycol) 15-hydroxystearate to propylene glycol is 1:5-5:1, preferably 1:3-3:1, more preferably 2:3.

**8.** A liquid injectable Plinabulin composition, comprising:

a D5W solution (5% dextrose injection) of Plinabulin, propylene glycol and poly(ethylene glycol) 15-hydroxystearate;
wherein the volume ratio of propylene glycol to D5W in the composition is about 6:50 to about 6:500.

**9.** The liquid injectable Plinabulin composition of claim 8, wherein the liquid injectable Plinabulin composition is prepared by a method comprising the following steps:

providing an initial liquid formulation comprising Plinabulin, propylene glycol and poly(ethylene glycol) 15-hydroxystearate; and
diluting the initial liquid formulation in D5W at a dilution ratio of about 1:5 to about 1:50;
wherein the initial liquid formulation is the Plinabulin micelle composition of claim 4.

**10.** The liquid injectable Plinabulin composition of claim 8, wherein the concentration of Plinabulin is about 0.08 mg/ml to about 0.4 mg/ml.

**11.** The liquid injectable Plinabulin composition of any one of claims 8-10, wherein the volume ratio of poly(ethylene glycol) 15-hydroxystearate to D5W in the liquid injectable Plinabulin formulation is about 4:50 to about 4:500, preferably about 4:100 to about 4:500, more preferably about 4:100 to about 4:400.

**12.** The liquid injectable Plinabulin composition of claim 8, wherein the total amount of impurities in the liquid injectable Plinabulin composition is less than 0.5%.

**13.** The liquid injectable Plinabulin composition of claim 8, wherein greater than about 90% of the Plinabulin in the composition is encapsulated in micelle.

**14.** A method for preparing a liquid injectable Plinabulin composition, wherein the method comprises:

providing an initial liquid formulation comprising Plinabulin, propylene glycol and poly(ethylene glycol) 15-hydroxystearate; and
diluting the initial liquid formulation in D5W at a dilution ratio of about 1:5 to about 1:50.

**15.** The method for preparing a liquid injectable Plinabulin composition of claim 14, wherein the initial liquid formulation is the Plinabulin micelle composition of claim 4.

**16.** Use of the Plinabulin micelle composition of claim 4 or the liquid injectable Plinabulin composition of claim 8 in the preparation of an anticancer drug.

**17.** The use of claim 16, wherein the cancer is selected from: lung cancer (such as small cell lung cancer, non-small cell lung cancer), prostate cancer, colon cancer, brain tumor (such as glioblastoma, polymorphic glioblastoma, giant cell glioblastoma, metastatic brain tumor), head and neck cancer, gastric cancer, pancreatic cancer, breast cancer, renal cancer, bladder cancer, ovarian cancer, cervical cancer, melanoma, myeloma, lymphoma or leukemia.

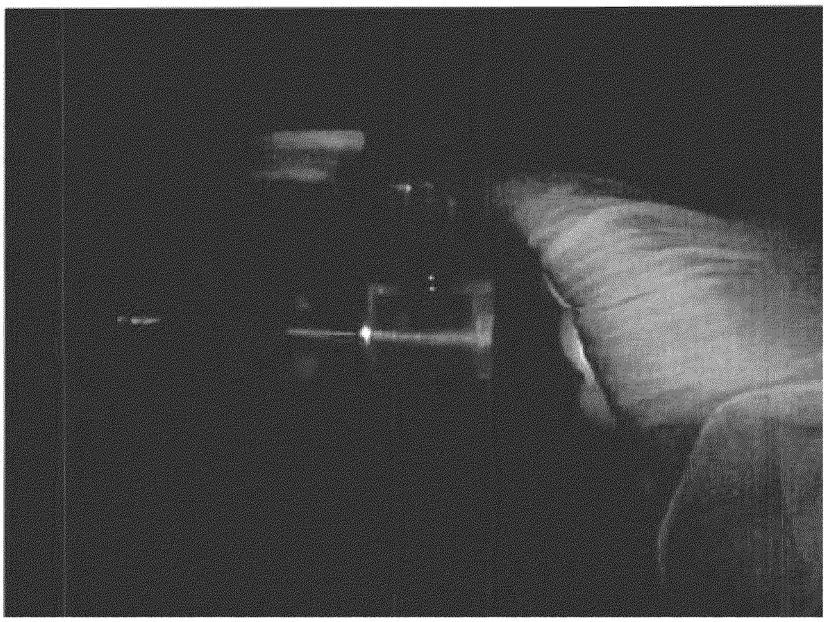

Figure 1

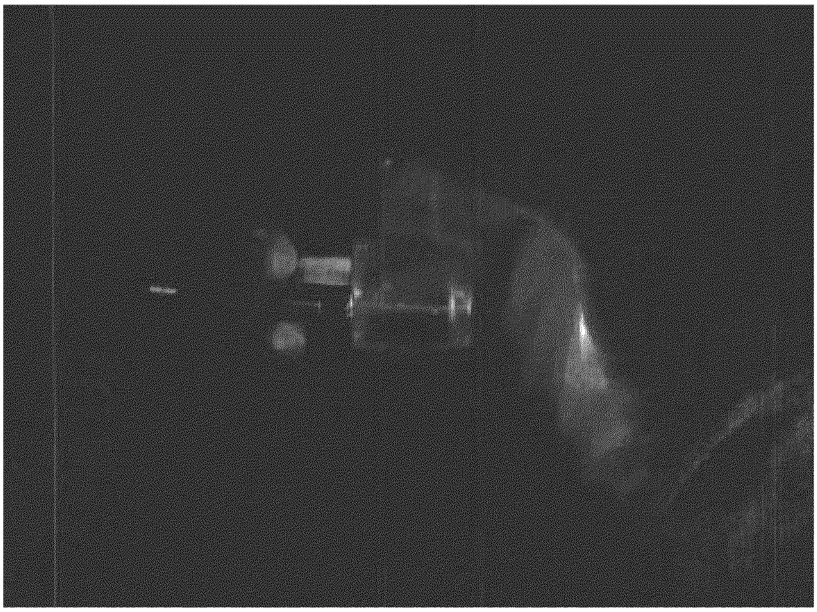

Figure 2

**INTERNATIONAL SEARCH REPORT**

International application No. **PCT/CN2023/124000**

**A. CLASSIFICATION OF SUBJECT MATTER**

A61K 9/127(2006.01)i; A61K31/496(2006.01)i; A61P35/00(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNABS, CNTXT, DWPI, WOTXT, EPTXT, USTXT, CNKI, 万方, WANGANG, 读秀, DUXIU, PUBMED, ISI_Web of Science, Science Direct, STNext: 大连万春, 黄岚, 何成江, 普那布林, 15-羟基硬脂酸聚乙二醇酯, 丙二醇, 胶束, NPI 2358, Plinabulin, NPI-2358, KPU-2, Kolliphor®# HS# 15, HS-15, polyoxyl 15 hydroxystearate, macrogol 15 hydroxystearate, propylene glycol, micella

**C. DOCUMENTS CONSIDERED TO BE RELEVANT**

| Category* | Citation of document, with indication, where appropriate, of the relevant passages | Relevant to claim No. |
|---|---|---|
| X | CN 110603037 A (DALIAN WANCHUNBULIN PHARMACEUTICAL CO., LTD.) 20 December 2019 (2019-12-20)<br>description, paragraph 0150 | 8, 14 |
| Y | CN 108026075 A (BEYONDSPRING PHARMACEUTICALS INC.) 11 May 2018 (2018-05-11)<br>description, paragraphs 0005, 0151, and 0190-0191 | 1-7, 9-13, 15-17 |
| Y | CN 110603037 A (DALIAN WANCHUNBULIN PHARMACEUTICAL CO., LTD.) 20 December 2019 (2019-12-20)<br>description, paragraph 0150 | 1-7, 9-13, 15-17 |
| Y | 张蕾 等 (ZHANG, Lei et al.). "非离子型表面活性剂HS 15 在药剂中的研究进展 (Research Progress of Nonionic Surfactant HS 15 in Pharmaceutical Preparations)"<br>*药学进展 (Progress in Pharmaceutical Sciences),* Vol. 39, No. 5, 31 May 2015 (2015-05-31), pages 370-375 | 1-7, 9-13, 15-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2023** | **19 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/124000**

**C. DOCUMENTS CONSIDERED TO BE RELEVANT**

| Category* | Citation of document, with indication, where appropriate, of the relevant passages | Relevant to claim No. |
|---|---|---|
| Y | CN 105705148 A (DALIAN WANCHUNBULIN PHARMACEUTICAL CO., LTD.) 22 June 2016 (2016-06-22)<br>description, paragraphs 0248-0249, and claims 1-10 | 1-7, 9-13, 15-17 |
| Y | CN 107530340 A (BEYONDSPRING PHARMACEUTICALS INC.) 02 January 2018 (2018-01-02)<br>description, paragraphs 0026 and 0073, and claims 1-13 | 1-7, 9-13, 15-17 |
| Y | CN 112543636 A (DALIAN WANCHUNBULIN PHARMACEUTICAL CO., LTD.) 23 March 2021 (2021-03-23)<br>description, paragraph 0029, and claims 1-22 | 1-7, 9-13, 15-17 |
| A | CN 109195584 A (CENTRE NATIONAL DE LA RECHERCHE SCIENTIFIQUE et al.) 11 January 2019 (2019-01-11)<br>description, paragraph 0093, and claims 1-14 | 1-17 |
| A | 辛景 (XIN, Jing). "多西紫杉醇聚合物胶束的应用研究进展 (Research Progress of Polymeric Micelles for Docetaxel Delivery)"<br>*价值工程 (Value Engineering)*, 31 December 2017 (2017-12-31),<br>pages 206-207 | 1-17 |
| A | CN 114796110 A (BEIJING DELI FURUI MEDICAL SCIENCE & TECHNOLOGY CO., LTD.) 29 July 2022 (2022-07-29)<br>claims 1-13 | 1-17 |

Form PCT/ISA/210 (second sheet) (July 2022)

**INTERNATIONAL SEARCH REPORT**
**Information on patent family members**

International application No.
**PCT/CN2023/124000**

| Patent document cited in search report | | | Publication date (day/month/year) | Patent family member(s) | | | Publication date (day/month/year) |
|---|---|---|---|---|---|---|---|
| CN | 110603037 | A | 20 December 2019 | NZ | 757213 | A | 28 January 2022 |
| | | | | WO | 2018169887 | A1 | 20 September 2018 |
| | | | | BR | 112019018880 | A2 | 14 April 2020 |
| | | | | EP | 4190326 | A1 | 07 June 2023 |
| | | | | DK | 3595653 | T3 | 17 April 2023 |
| | | | | KR | 20190122822 | A | 30 October 2019 |
| | | | | US | 2020129504 | A1 | 30 April 2020 |
| | | | | JP | 2023099217 | A | 11 July 2023 |
| | | | | JP | 2020520889 | A | 16 July 2020 |
| | | | | JP | 7280192 | B2 | 23 May 2023 |
| | | | | AU | 2018236168 | A1 | 10 October 2019 |
| | | | | EP | 3595653 | A1 | 22 January 2020 |
| | | | | EP | 3595653 | A4 | 11 November 2020 |
| | | | | EP | 3595653 | B1 | 08 March 2023 |
| | | | | SG | 11201908420 | WA | 30 October 2019 |
| | | | | CA | 3056077 | A1 | 20 September 2018 |
| | | | | ES | 2942889 | T3 | 07 June 2023 |
| CN | 108026075 | A | 11 May 2018 | ECSP | 18010481 | A | 30 April 2018 |
| | | | | AU | 2021201784 | A1 | 15 April 2021 |
| | | | | AU | 2021201784 | B2 | 01 December 2022 |
| | | | | MY | 181892 | A | 12 January 2021 |
| | | | | EP | 4011878 | A1 | 15 June 2022 |
| | | | | WO | 2017011399 | A1 | 19 January 2017 |
| | | | | CA | 2991059 | A1 | 19 January 2017 |
| | | | | ES | 2910035 | T3 | 11 May 2022 |
| | | | | EP | 3334726 | A1 | 20 June 2018 |
| | | | | EP | 3334726 | A4 | 17 April 2019 |
| | | | | EP | 3334726 | B1 | 16 March 2022 |
| | | | | JP | 2021181497 | A | 25 November 2021 |
| | | | | US | 2022169635 | A1 | 02 June 2022 |
| | | | | PE | 28 May 2018 | A1 | 19 March 2018 |
| | | | | US | 2019106410 | A1 | 11 April 2019 |
| | | | | US | 10550104 | B2 | 04 February 2020 |
| | | | | AU | 2016291708 | A1 | 01 February 2018 |
| | | | | AU | 2016291708 | B2 | 24 December 2020 |
| | | | | KR | 20180027563 | A | 14 March 2018 |
| | | | | JP | 2018520178 | A | 26 July 2018 |
| | | | | JP | 6969848 | B2 | 24 November 2021 |
| | | | | HK | 1251559 | A1 | 01 February 2019 |
| | | | | MX | 2018000451 | A | 07 May 2018 |
| | | | | IL | 256559 | A | 28 February 2018 |
| | | | | IL | 256559 | B1 | 01 March 2023 |
| | | | | IL | 256559 | B2 | 01 July 2023 |
| | | | | BR | 112018000229 | A2 | 04 September 2018 |
| | | | | US | 2020277280 | A1 | 03 September 2020 |
| | | | | US | 11254657 | B2 | 22 February 2022 |
| | | | | US | 2018194749 | A1 | 12 July 2018 |
| | | | | US | 10155748 | B2 | 18 December 2018 |
| | | | | CL | 2018000098 | A1 | 20 July 2018 |
| | | | | SG | 10202108194 | XA | 29 September 2021 |

Form PCT/ISA/210 (patent family annex) (July 2022)

**INTERNATIONAL SEARCH REPORT**
**Information on patent family members**

International application No.
**PCT/CN2023/124000**

| Patent document cited in search report | Publication date (day/month/year) | Patent family member(s) | Publication date (day/month/year) |
|---|---|---|---|
| | | DK 3334726 T3 | 16 May 2022 |
| | | CO 2018000350 A2 | 12 June 2018 |
| | | PH 12018500071 B1 | 23 July 2018 |
| CN 105705148 A | 22 June 2016 | DK 3076972 T3 | 03 January 2022 |
| | | RU 2016112608 A | 16 November 2017 |
| | | RU 2662298 C2 | 25 July 2018 |
| | | NZ 719049 A | 26 June 2020 |
| | | CA 2926771 A1 | 16 April 2015 |
| | | CA 2926771 C | 05 July 2022 |
| | | ES 2902665 T3 | 29 March 2022 |
| | | US 2016250209 A1 | 01 September 2016 |
| | | US 10596169 B2 | 24 March 2020 |
| | | JP 2016536352 A | 24 November 2016 |
| | | JP 6411523 B2 | 24 October 2018 |
| | | WO 2015051543 A1 | 16 April 2015 |
| | | WO 2015051543 A8 | 21 April 2016 |
| | | SG 11201602637 QA | 30 May 2016 |
| | | KR 20160078987 A | 05 July 2016 |
| | | KR 102225371 B1 | 10 March 2021 |
| | | AU 2013402794 A1 | 12 May 2016 |
| | | AU 2013402794 B2 | 27 February 2020 |
| | | US 2020281921 A1 | 10 September 2020 |
| | | ZA 201602380 B | 25 May 2022 |
| | | BR 112016007946 A2 | 12 September 2017 |
| | | MY 185650 A | 27 May 2021 |
| | | IL 244984 A0 | 31 May 2016 |
| | | IL 244984 B | 01 May 2022 |
| | | EP 3076972 A1 | 12 October 2016 |
| | | EP 3076972 A4 | 20 December 2017 |
| | | EP 3076972 B1 | 06 October 2021 |
| | | MX 2016004441 A | 28 October 2016 |
| CN 107530340 A | 02 January 2018 | US 2018042921 A1 | 15 February 2018 |
| | | US 10076518 B2 | 18 September 2018 |
| | | CL 2017002242 A1 | 02 April 2018 |
| | | SG 11201707128 TA | 28 September 2017 |
| | | AU 2016229295 A1 | 28 September 2017 |
| | | AU 2016229295 B2 | 04 November 2021 |
| | | WO 2016144636 A1 | 15 September 2016 |
| | | HK 1249051 A1 | 26 October 2018 |
| | | EP 3265091 A1 | 10 January 2018 |
| | | EP 3265091 A4 | 01 August 2018 |
| | | MX 2017011375 A | 23 January 2018 |
| | | MY 194341 A | 29 November 2022 |
| | | KR 20170123642 A | 08 November 2017 |
| | | IL 254262 A0 | 31 October 2017 |
| | | RU 2017131448 A | 08 April 2019 |
| | | RU 2017131448 A3 | 20 August 2019 |
| | | RU 2728796 C2 | 31 July 2020 |
| | | US 2019000841 A1 | 03 January 2019 |
| | | US 10357491 B2 | 23 July 2019 |

Form PCT/ISA/210 (patent family annex) (July 2022)

**INTERNATIONAL SEARCH REPORT**
**Information on patent family members**

International application No.
**PCT/CN2023/124000**

| Patent document cited in search report | Publication date (day/month/year) | Patent family member(s) | Publication date (day/month/year) |
|---|---|---|---|
| | | AU 2021254549 A1 | 18 November 2021 |
| | | BR 112017018964 A2 | 22 May 2018 |
| | | JP 2018507245 A | 15 March 2018 |
| | | JP 6904570 B2 | 21 July 2021 |
| | | ZA 201706035 B | 18 December 2019 |
| | | JP 2021091695 A | 17 June 2021 |
| | | JP 7264331 B2 | 25 April 2023 |
| | | CA 2978679 A1 | 15 September 2016 |
| | | HK 1250007 A1 | 23 November 2018 |
| CN 112543636 A | 23 March 2021 | EP 3801524 A1 | 14 April 2021 |
| | | EP 3801524 A4 | 02 March 2022 |
| | | US 2021161888 A1 | 03 June 2021 |
| | | AU 2019278886 A1 | 24 December 2020 |
| | | CA 3101612 A1 | 05 December 2019 |
| | | MX 2020012799 A | 25 March 2021 |
| | | JP 2021525768 A | 27 September 2021 |
| | | WO 2019232257 A1 | 05 December 2019 |
| | | KR 20210015875 A | 10 February 2021 |
| CN 109195584 A | 11 January 2019 | CA 2998541 A1 | 23 March 2017 |
| | | JP 2018528970 A | 04 October 2018 |
| | | EP 3349725 A1 | 25 July 2018 |
| | | WO 2017046369 A1 | 23 March 2017 |
| | | AU 2016323727 A1 | 12 April 2018 |
| | | US 2019192430 A1 | 27 June 2019 |
| | | US 11291630 B2 | 05 April 2022 |
| CN 114796110 A | 29 July 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 113735834 B
- US 7064201 B
- US 7919497 B
- US 10238650 B
- US 10155748 B
- US 10076518 B
- US 10596169 B
- WO 2016130839 A
- WO 2017214052 A
- WO 2018144764 A
- WO 2018169887 A
- WO 2019147615 A
- WO 2019152530 A
- WO 2020037285 A
- WO 2021076485 A
- WO 2021225908 A